# EUROPEAN PATENT SPECIFICATION

(11) **EP 0 852 482 B1**
(45) Date of publication and mention of the grant of the patent: **31.10.2001**
(21) Application number: 96929061.8
(22) Date of filing: 20.08.1996
(51) Int. Cl.: A61F 13/15

(54) **ABSORBENT ARTICLE HAVING COMPLIANT CUFFS**
ABSORPTIONSFÄHIGES PRODUKT MIT EINER WEICHSTOFFDICHTUNG
ARTICLE ABSORBANT A FRONCES ELASTIQUES

(30) Priority: 01.09.1995 US 522876
(43) Date of publication of application: 15.07.1998
(73) Proprietor: McNEIL-PPC, Inc., Skillman, New Jersey 08558 (US)
(72) Inventor: SALERNO, Catherine, E., Malabar, New South Wales 2036 (AU); JERSCHKOW, Tenny, Middletown, NJ 07748 (US); LEMLI, Barbara, Kendall Park, NJ 08824 (US); McCOY, Sherilyn, Skillman, NJ 08558 (US)
(74) Representative: Mercer, Christopher Paul
(86) International application number: US9613729
(87) International publication number: WO9709016

(56) References cited:
- EP-A- 0 203 712
- EP-A- 0 534 488
- EP-A- 0 685 212
- WO-A-93/12747
- GB-A- 2 289 418
- US-A- 4 029 101
- US-A- 4 608 047

## Description

### Field of the Invention

The present invention relates an absorbent article for use in the perineal area of the body, such as sanitary napkins, incontinence pads, and the like, and more specifically relates to an absorbent article having compliant side cuffs along the lateral edges of the absorbent article that provide enhanced conformability of the absorbent article to the wearer's body, thereby inhibiting or preventing leakage past the lateral sides of the absorbent article.

### Background of the Invention

Traditional absorbent articles are generally characterized as having a body fluid pervious material defining a body facing side, a body fluid impervious material defining a garment facing side, and a central absorbent core between the body facing side and the garment facing side. The body facing side layer and the garment facing side layer are generally sealed around the outer edge margins of the central absorbent core so as to form laterally extending flanges (see, for example U.S. Patent No. 4,678,527 to Ulman).

Under certain circumstances, absorbent articles are subject to lateral leakage, for example, if the article is locally not in contact with the perineum because of wrinkling or deformation of the absorbent article or if the flow of body fluids exceeds the local absorbent capacity of the absorbent article. Such lateral leakage causes fluid to flow along and beyond the surface of the perineum to the user's legs often resulting in soiling of the undergarment or other clothing articles. Attempts have been made to enhance the fit of such absorbent products by imparting an arcuate shape to the article in the longitudinal direction. This is typically accomplished by applying longitudinally extending elastic elements placed in tension to the article (see, for example U.S. Patent Nos. 3,236,238 to Morse and 4,432,823 to Moore).

U.S. Patent No. 4,701,177 to Ellis et al. discloses a sanitary napkin in which the absorbent core in the central portion of the napkin has a dog bone shape, i.e., the width of the absorbent core is reduced in the central portion. The cover layer and barrier layer are sealed along their lateral edges in a contour which follows the top edges of the lateral sides of the central absorbent core. As a result, in the reduced width central portion of the napkin, there are portions of the cover and barrier which are outward of the absorbent core which tend to form walls. Elastic members disposed within the walls cause them to extend upward above the body facing surface and into the crease at the sides of the pudendum so as to prevent leakage. However, this arrangement suffers from several drawbacks.

First, as a result of the placement of the seal lines at the top of the sides of the central portion, the walls have a tendency to fold inward about the joint lines in use so that they lay over the body facing surface, thereby reducing the effective area of the central absorbent portion. Second, the formation of walls requires that the central portion be formed into a dog bone shape so that this sealing approach is not applicable to all types of napkins. Third, the materials that form the walls are limited to those suitable for napkin covers and barriers. Finally, since the perineal area of the body does not have an arcuate or cup shape, these products are not fully effective for maintaining contact between the perineal area and the absorbent product.

Other attempts to prevent lateral side leakage in absorbent articles have been to provide wings or flaps which wrap around the crotch portion of the wearer's undergarment to cover and thus protect the undergarment. For example, U.S. Patent No. 4,285,343 to McNair or U.S. Patent No. 4,589,876 to Van Tilburg disclose sanitary napkins having wings in which flexible axes are formed that allow the wings to be folded over the edges of the panty crotch. In Van Tilburg, each wing is joined to the central portion of the napkin along a preferential bending line. The width of the central portion is less than the span of the perineum so that the elastic in the panty crotch bends the wings upward around the preferential bending line. This bending action causes the wings to form walls that bear against the laterally outward surfaces of the perineum to produce a seal that is described as being gasket-like. Unfortunately, such articles suffer from several drawbacks.

First, since the article relies on the panty elastic to bend the wings upward around the laterally outward surfaces of the perineum at the preferential bending joints between the wings and the central portion, the maximum width of the central portion is limited to the width of the panty crotch. This limits the absorbent capacity of the napkin as well as its applicability to a large variety of panty sizes.

Second, since the wings are folded over the edges of the panty crotch, the seals formed thereby can extend beyond the edges of the crotch only by the thickness of the wings. As a result, optimal contact of the seal with the body will not be attained for all users since the seal does not extend a substantial distance beyond the edge of the crotch. Again, this limits the applicability of the napkin.

Third, although the preferential bending line and flexible axis give the wing flexibility in the direction normal to the plane of the wing, the wing is relatively stiff in response to a compression force applied in the plane of the wing. Hence, the compliancy of the wings is low, resulting in discomfort due to the wings digging into the body.

Fourth, since the elastic portion of the crotch is disposed at the top of the wall formed by the wing, the force imposed by deformation of the elastic portion acts to press only the wing against the user's body. The elastic portion does not push the central portion of the article against the perineum so as to ensure proper contact.

Absorbent articles in which the layers are joined by forming flanges, as discussed above, suffer from the drawback that although the flanges are flexible with respect to forces acting perpendicular to the plane of the flange, they are fairly rigid in response to forces acting in-plane. Consequently, the flanges of such articles have a tendency to dig into the skin of the user, causing discomfort.

U.S. Patent No. 4,695,278 to Lawson discloses a diaper in which flaps are formed by extending the cover and barrier beyond the sides of the central portion and joining them together along longitudinally extending joint lines spaced transversely from the central portion sides. Elastic members are disposed within the flaps, to form members characterized as "barrier cuffs." Members characterized as barrier cuffs are formed by attaching strips of material, folded over so as to form loops at their distal ends, to the flaps along the joint lines. Elastic members are disposed within the loops causing them to extend vertically upward above the body facing surface. Unfortunately, as a result of the length of the barrier cuffs and the spacing of the joint lines away from the sides of the central portion, the barrier cuffs, like the walls in the Ellis patent, have a tendency in use to fold inward about the joint lines so that they lay over the body facing surface, thereby reducing the effective area of the central absorbent portion.

European Patent Application EP A 0 534 488 discloses an absorbent article having compliant gaskets along the longitudinal sides of the article. WO 93/12747 discloses an absorbent article having elasticized side flaps.

Consequently, it is desirable to provide an absorbent article that overcomes the aforementioned drawbacks associated with absorbent articles heretofore known in the art. Such an article should be capable of preventing lateral leakage of fluid; maintain body contact with the effective area of the central absorbent; be adapted to fit properly regardless of the size of the user or the undergarment; be sufficiently compliant to provide a comfortable fit and have the surface properties which minimize any unpleasant sensation of such contact while ensuring a good fit and proper contact of the central portion of the absorbent article with the body.

### Summary of the Invention

It is an object of the current invention to provide an absorbent article which inhibits or prevents leakage of fluid past the lateral edges of the absorbent article.

It is another object of the current invention to provide an absorbent article which is suitable for use in undergarments having a large range of sizes.

It is yet another object to provide an absorbent article having compliant side cuffs which make good sealing contact with the user's body and yet are comfortable.

In accordance with the present invention, these and other objects have been provided in an absorbent article for use in the perineal area of a user's body to absorb body fluid, said absorbent article having right and left lateral sides and first and second transverse ends, said absorbent article comprising:
a) a fluid pervious first layer forming a body facing surface;
b) a fluid impervious second layer forming a garment facing surface opposite the body facing surface;
c) an absorbent core positioned between the first layer and the second layer;
d) right and left longitudinally extending cuffs, the cuffs having a base portion and a distal end, each of the cuffs comprising a longitudinal strip of a resilient, highloft, fluid permeable material which is covered, at least in part, with a flexible, fluid repellent porous material, and wherein the right and left longitudinally extending cuffs are attached along their respective base portions to the right and left lateral sides of the absorbent article, respectively, such that the distal ends of the cuffs extend outward from the right and left lateral sides of the absorbent article.

Also provided in accordance with the present invention is an absorbent article for absorbing fluid in the perineal area of the user's body and adapted for use in conjunction with an undergarment having a crotch portion having right and left edges, the absorbent article comprising:
a) a fluid pervious first layer forming a body facing surface, a fluid impervious second layer forming a garment facing surface opposite the body facing surface, an absorbent core between the first and second layers and having right and left longitudinally extending opposing sides, the first and second layers having first joining means for joining the first and second layers, thereby forming right and left flanges adjacent the right and left sides of the absorbent core, respectively;
b) right and left longitudinally extending cuffs, each cuff comprising a longitudinal strip of a resilient, highloft, fluid permeable material which is covered, at least in part, with a flexible, fluid repellent porous material, each cuff having a base portion and a distal end, wherein each cuff is attached along its respective base portion to the absorbent article adjacent the right and left flanges and wherein the distal end of each cuff extends outward therefrom, whereby the distal ends of the cuffs are compressed against a portion of the user's body in use;
c) right and left wings each having a base portion and a distal end portion, the base portion being attached to the fluid impervious layer inwardly from the lateral edges of the absorbent article, thereby forming right and left pockets, the distal end portion adapted to fold over and retain the right and left edges of the undergarment crotch portion in the right and left pockets, respectively.

Also provided in accordance with the present invention is an absorbent article for use in the perineal area of a user's body to absorb body fluid, the absorbent article having right and left lateral sides and first and second transverse ends, the absorbent article comprising:
a) a fluid pervious first layer forming a body facing surface;
b) a fluid impervious second layer forming a garment facing surface opposite the body facing surface;
c) an absorbent core positioned between the first layer and the second layer wherein the first layer has right and left approximately longitudinally extending edges, and the second layer has right and left approximately longitudinally extending edges, and wherein the first layer is joined to the second layer adjacent to their respective right and left edges so as to form right and left flanges, wherein the right and left flanges are adjacent the right and left lateral sides of the absorbent article;
d) right and left longitudinally extending cuffs, the cuffs having a base portion and a distal end, each of the cuffs comprising a longitudinal strip of a resilient, fluid permeable, highloft material, and a longitudinal strip of an apertured polymeric film which encloses the highloft material along at least a substantial portion of its length and wherein the right and left longitudinally extending cuffs are adhered along their respective base portions to the right and left lateral sides of the absorbent article, respectively, such that the distal ends of the cuffs extend outward from the right and left flanges;
e) right and left wings each having a base portion and a distal end portion, the right and left wings attached at their respective base portions to the right and left flanges, respectively, and forming a part thereof, and the distal ends being foldable over a crotch portion of a user's undergarment.

### Brief Description of the Drawings

Figure 1 is an isometric view of one embodiment of an absorbent article according to the current invention.
Figure 2 is a plan view of the article shown in Figure 1.
Figure 3 is an elevation view of the article shown in Figure 1.
Figure 4 is a transverse cross-section through the article shown in Figure 1 taken through line IV-IV.
Figure 5 is a transverse cross-section through the article shown in Figure 1 taken through line V-V.
Figures 6 and 7 are detailed views of embodiments of the portion of the article shown in Figure 5 enclosed by the circle VI.
Figure 8 is a transverse cross-section through the embodiment of the article shown in Figure 1 in use.
Figure 9 is a transverse cross-section through a another embodiment of the article shown in Figure 1.
Figure 10 is a transverse cross-section through a still another embodiment of the article shown in Figure 1.
Figures 11 (a)-(d) are detailed views of embodiments of the portion of the article shown in Figure 10 enclosed by the circle X.
Figure 12 is a transverse cross-section through a still another embodiment of the article shown in Figure 1.
Figures 13 (a)-(c) are detailed views of embodiments of the portion of the article shown in Figure 12 enclosed by the circle XIII.
Figure 14 is an isometric view of yet another embodiment of an absorbent article according to the current invention having wings.
Figure 15 is an elevation view of the article shown in Figure 14.
Figure 16 is a transverse cross-section through the article shown in Figure 14 taken through line XVI-XVI.
Figures 17 (a)-(c) are detailed views embodiments of the portion of the article shown in Figure 16 enclosed by the circle XVII.
Figure 18 is a transverse cross-section through the article shown in Figure 14 in use.
Figure 19 is a plane view from below of the embodiment of the article shown in Figure 14 in use.
Figure 20 is a transverse cross-section through another embodiment of the article shown in Figure 14 in use.
Figure 21 shows an alternate embodiment of the cuff shown in Figure 6.
Figure 22 shows two alternate embodiments of the cuff shown in Figure 13.

### Description of the Preferred Embodiments

The present invention is directed to an absorbent article for use in the perineal area of a user's body to absorb body fluid. The absorbent article has right and left lateral sides and first and second transverse ends and comprises: a) a fluid pervious first layer forming a body facing surface; b) a fluid impervious second layer forming a second surface opposite the body facing surface; c) an absorbent core positioned between the first layer and the second layer; d) right and left longitudinally extending compliant cuffs.

The cuffs comprise a longitudinal strip of high loft material and a longitudinal strip of fluid repellent porous material which encloses the high loft material along at least a substantial portion of its length. The compliant cuffs of the present invention have a base portion and a distal end portion, and are attached along a substantial portion of their respective base portions to the right and left lateral sides of the absorbent article, respectively, such that the distal end portions of the cuffs extend outward from the right and left lateral sides of the absorbent article.

The strip of high loft material 47 may be formed from a fibrous woven or nonwoven flexible fabric that is soft, comfortable, and cushiony,to provide a comfortable "feel" to the user, and also possesses an open, fluid-permeable structure, i.e., having void spaces which are capable of holding or retaining fluid, and which may optionally be capable of drawing fluid away from the fluid repellent porous material which covers the high loft material to provide a 'clean-dry' appearance to the cuff. As used herein the term 'high loft material' refers to materials which either as a single layer or as a multi-layer laminate provide a total thickness of the cuff of at least 0.635mm (25 mils). The exact thickness of either the high loft material or the apertured polymeric film is not per se critical to the invention, provided of course, that the total thickness of the cuff is at least 0.635mm (25 mils), preferably at least 1.02mm (40 mils), and most preferably between 1.02mm-2.032mm(40 and 80 mils). The strip of high loft material 48 may be wicking or non-wicking, and is preferably non-wicking so as not to promote the flow of fluid beyond the cuff 6. The resilient, fluid permeable high loft material is preferably flexure resistant. Flexure resistance is generally measured by peak bending stiffness which is more fully discussed in U.S. patent 5,171,302 to Buell, which is incorporated herein in its entirety. The high loft material of the present invention preferably has a peak bending stiffness of at least 100 grams, preferably greater than 200 grams, and most preferably between 250 grams and 1000 grams.

Suitable resilient, fluid permeable high loft materials include, but are not limited to, high loft polyesters, polyethylene/polyester bi-component fibers, wood pulp such as air laid pulps, wet laid pulps, and the like, pulp-fiber blends, laminates of the above materials or further laminated to polymeric films, with the proviso that the upper-most layer in the laminate structure, (which corresponds to the body-facing side of the absorbent article) possesses an open, fluid permeable structure capable of holding or retaining fluid, and combinations of the above materials. A particularly preferred material is a fusible fiber which is commercially available under the trademark ENKA™ having a bulk density of about 18.6 g/sq.m (0.5 oz./sq. yd.) and a thickness of about 0.22mm (9 mils).

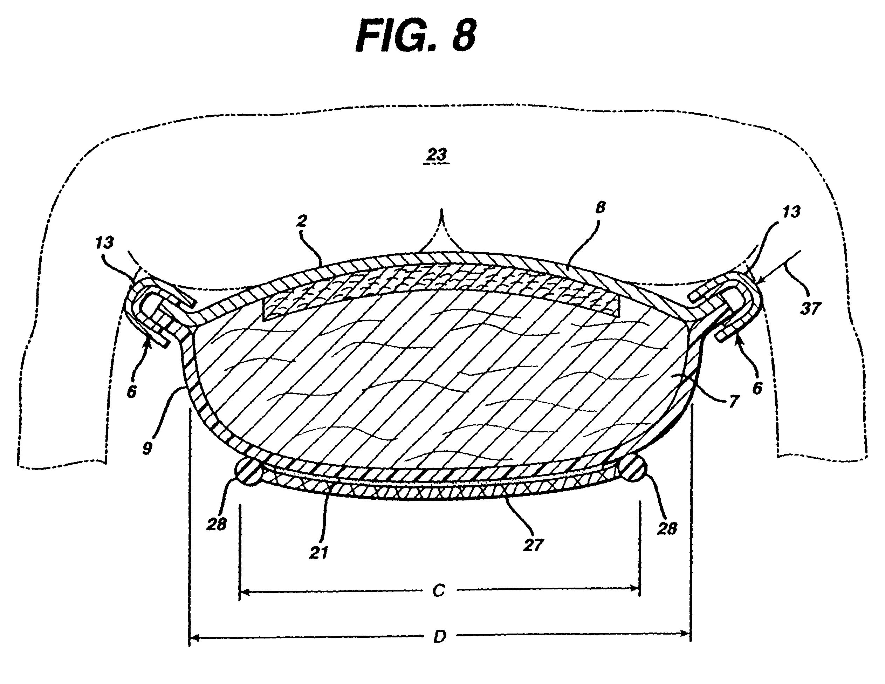

In addition to a comfortable feel, the cuffs preferably provide a smooth, curved, edgeless surface at their distal ends that serves as the contact surface for the cuff against the body, as shown in Figure 8. Such contouring of the cuff contact surface minimizes any discomfort associated with such contact under pressure.

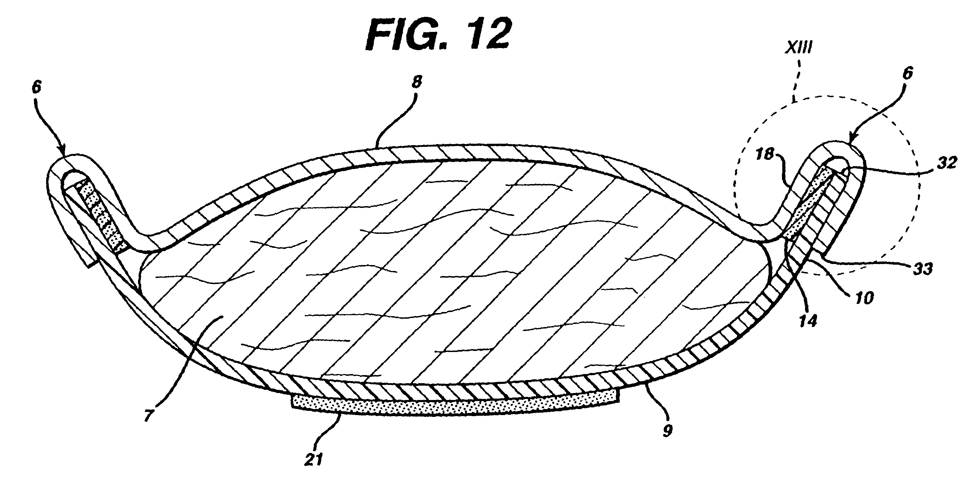

Suitable materials for use as the fluid repellent porous material include, but are not limited to, any of the conventional fluid repellent materials used as fluid pervious cover layers in commercially available absorbent sanitary products as discussed for fully below. A preferred material for use as the fluid repellent porous material is an apertured polymeric film. A particularly preferred apertured polymeric film which may be used both as the cover layer and as the fluid repellent porous material to cover the high loft material of the cuff is disclosed in Canadian Patent Application 2,130,176, which is incorporated herein in its entirety. In a most preferred embodiment, the apertured polymeric film is applied as a separate strip of material which is attached to the side cuffs while ' the film is slightly tensioned. Alternatively, it is the film is slightly tensioned. Alternatively, it is also possible to use the same apertured polymeric film which was used as the cover layer to simultaneously form the side cuffs as shown in Figure 12 - 13.

The surface 16 of the absorbent article that is intended to be worn against the body of the user (i.e the body facing surface) is covered by a layer 8 of a body-fluid pervious material, typically referred to as a "cover layer". The cover layer 8 may be formed from any fluid pervious material that is comfortable against the skin and that permits fluid to penetrate to the underlying core 7, which retains the fluid. The cover layer 8 is preferably fluid repellent, i.e. it should retain little or no fluid in its structure to provide a relatively dry surface next to the skin. The fluid pervious cover layer 8 may be a fibrous woven or nonwoven fabric made of fibers or filaments of polymers such as cotton, rayon, nylon, polyethylene, polypropylene, polyester, bi-component fibers of polyester sheathed in polyethylene or polypropylene, bi-component fibers of polypropylene sheathed in polyethylene, fibers of high melting polyester sheathed in low melting polyester, and combinations thereof.

In a most preferred embodiment, the cover layer 8 may be formed from an apertured polymeric film. The thickness of the cover layer 8 will vary from approximately 0.254 to 1.57mm (0.001 to 0.062 inch), preferably from 0.127 to 0.57mm (0.005 to 0.020 inch) and most preferably from 0.254 to 0.381mm (0.010 to 0.015 inch). Generally, the fluid pervious cover layer 8 is a single sheet of material having a surface area sufficient to cover the body-facing surface 16 of the absorbent article. Preferably, the fluid pervious cover layer 8 is longer than the core 7 so as to form the transverse ends 3. The transverse ends 3 may be sealed with other pervious or non-pervious layers to fully enclose the central absorbent core.

The absorbent article 1 further comprises a layer 9 of a body fluid impervious material, typically referred to as a "barrier layer", on its garment facing surface 17. The fluid impervious barrier layer 9 may comprise any thin, flexible, body fluid impermeable material and is typically a polymeric film such as, for example, polyethylene, polypropylene, cellophane, and the like. Alternatively, the barrier layer may be a normally fluid pervious material that has been treated to be impervious, such as impregnated fluid repellent paper, woven or nonwoven fabric material, closed cell flexible foams, such as polyurethane or cross-linked polyethylene. The fluid impervious barrier layer may optionally be permeable to gas including water vapor to provide an air breathable fluid barrier. The thickness of the barrier layer is not per se critical to the invention, provided of course that it is flexible. A typical barrier layer thickness, when formed from a polymeric film, is in the range of from 0.0127 to 0.635 mm (0.0005 to 0.025 inch) and is preferably in the range 0.254 to 0.508mm ( 0.001 to 0.002 inch).

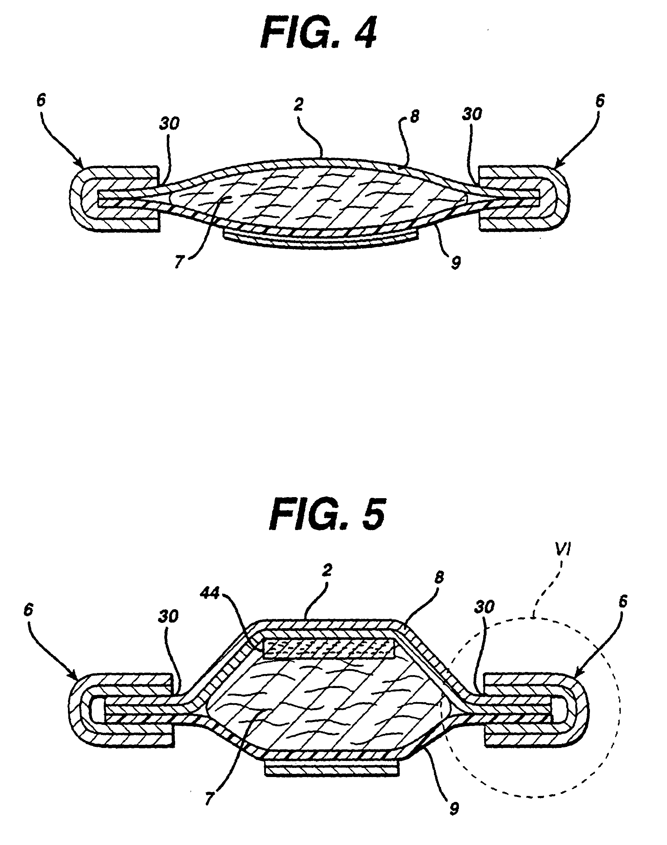

The barrier layer 9 generally comprises a single sheet of material having a surface area sufficient to cover the entire garment-facing surface 17 of the absorbent article. As shown in Figures 4-9, the fluid impervious barrier layer 9 may extend around the sides of the core 7 in a C-shaped configuration with the portions 10 of the barrier layer that are adjacent its longitudinal edges 32 extending upwardly from the garment facing surface 17 toward the body facing surface 16 so as to form a portion of the lateral sides 30 of the absorbent article 1.

The construction the absorbent article 1 is shown in Figures 4 and 5. The central portion 2 of the absorbent article contains an absorbent core 7. As is known in the art, the absorbent core 7 may be comprised of a loosely associated absorbent hydrophilic material such as cellulose fibers, including wood pulp, wet or dry cross-linked wood pulp, curly wood pulp fibers, rayon fibers or cotton fibers, or other absorbent materials generally known in the art, including acrylate fibers, polyvinyl alcohol fibers, peat moss or superabsorbent particles or fibers. The absorbent core may also comprise synthetic fibers such as nylon, polyethylene, polypropylene, polyester, bi-component fibers of polyester sheathed in polyethylene or polypropylene, bi-component fibers of polypropylene sheathed in polyethylene, fibers of high melt polyester sheathed in low melt polyester, and combinations thereof. These fibers may be layered to form an absorbent core having a porosity gradient, and/or may be treated with a surfactant to make them hydrophilic and thereby wettable.

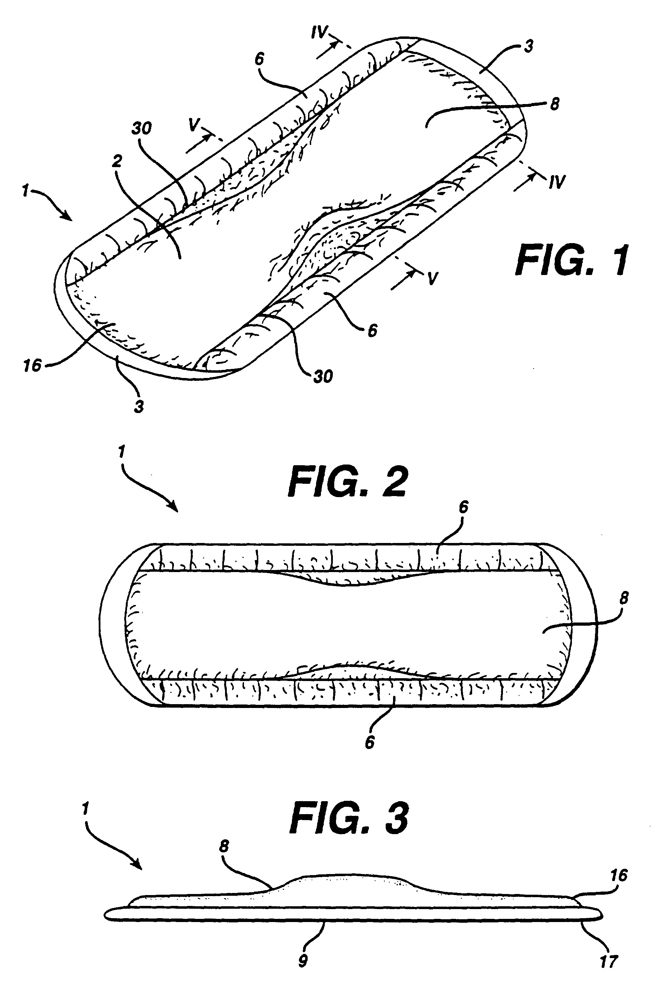

In a preferred embodiment, the central portion of the absorbent core has an hour glass shape and is thicker (i.e. in the "z" direction) in its center region than at its end portions, thus creating an absorbent article having a raised center portion, as shown in Figures 1 - 5. The relative thickness of the center region is not, per se, critical to the invention, and is generally from 1.1 to 4 times thicker than the transverse end regions of the absorbent core, preferably from 1.5 to 3 times thicker, and most preferably from 1.75 to 2.75 times thicker. The raised center portion has been found to provide enhanced body contact with the perineal area of the user's body, and thus enhances the transference of fluid from the user's body into the central absorbent core. In a most preferred embodiment, the raised center portion of the absorbent core further comprises an absorbent structure derived from sphagnum moss 44. Absorbent structures derived from sphagnum moss are more fully disclosed in U.S. Patent numbers 4,170,515 to Lalancette et al., 4,215,692, 4,226,237, and 4,507,122 to Levesque, 4,305,393 to Nguyen, 4,473,440 to Ovens, 4,618,496 to Brasseur, 4,676,871 to Cadieux et al., 4,992,324 to Dube, and 5,053,029 to Yang which are incorporated by reference herein in their entirety.

The absorbent article of the present invention may optionally include a transfer layer between the fluid pervious cover layer and the absorbent core. The transfer layer generally comprises similar materials as those used in the absorbent core, but will have a more open porous structure than the central absorbent core having interstitial spaces between the fibrous materials which are greater than those in the absorbent core. This creates a porosity gradient which greatly enhances the rate of fluid transfer form the cover layer to the absorbent core which acts as a reservoir for the absorbed fluids.

There is shown in Figures 1-3 one embodiment of the current invention illustrated with respect to an absorbent article 1 according to the current invention. The absorbent article is comprised of a longitudinally extending central portion 2 having longitudinal sides 30 and transverse ends 3. The central portion may have an approximately rectangular shape, an approximately oval shape, or preferably an hour-glass or dog-bone shape wherein the transverse ends are wider than the central portion of the absorbent article.

As shown best in Figures 1, 4, and 5, right and left hand longitudinally extending cuffs 6 are attached at their bases to the lateral sides 30 of the central portion 2. According to the current invention, the cuffs 6 are in a general longitudinal rectangular configuration so as to form substantially parallel cuffs along the lateral sides of the absorbent article. As shown in Figures 1 and 2, the cuffs extend substantially the entire length of the absorbent article. While the length of the cuffs is not, per se, critical to the invention, it is important that the cuffs extend at least across the central portion of the absorbent article, i.e., in the area of the absorbent article which will be adjacent to the perineal area of a user. Accordingly, the length of the cuffs will generally extend at least 50% of the absorbent article length, preferably the cuffs extend at least 75% of the absorbent article length, and most preferably the cuffs extend the entire length of the absorbent article.

There are a number of factors which are considered important in determining the width of the compliant cuffs of the present invention. One factor is that the width of the absorbent article should be at least as wide as the width of the crotch portion of a user's panty. These widths can vary widely and are generally in the range of from about 6.35 to 10.16cm (2.5" to about 4"). Another factor to consider is the width of the central absorbent core. The central absorbent core should be wide enough to assure a good fit against the perineal area of the user but should not be wider than the perineal area of the user since this may lead to discomfort due to rubbing and/or chafing of the inner thighs of the user. While the anatomy of the perineal area can vary widely, it has generally been found that the central absorbent cores can have widths in the range of from about 5.1 to 7.62cm (2" to 3"). In consideration of the foregoing factors, the compliant cuffs of the present invention will accordingly have widths in the range of from about 0.635 to 2.54cm (0.25" to about 1"), preferably in the range of about 0.762 to 2.03cm (0.3" to about 0.8"), and most preferably about 1.9cm (.75").

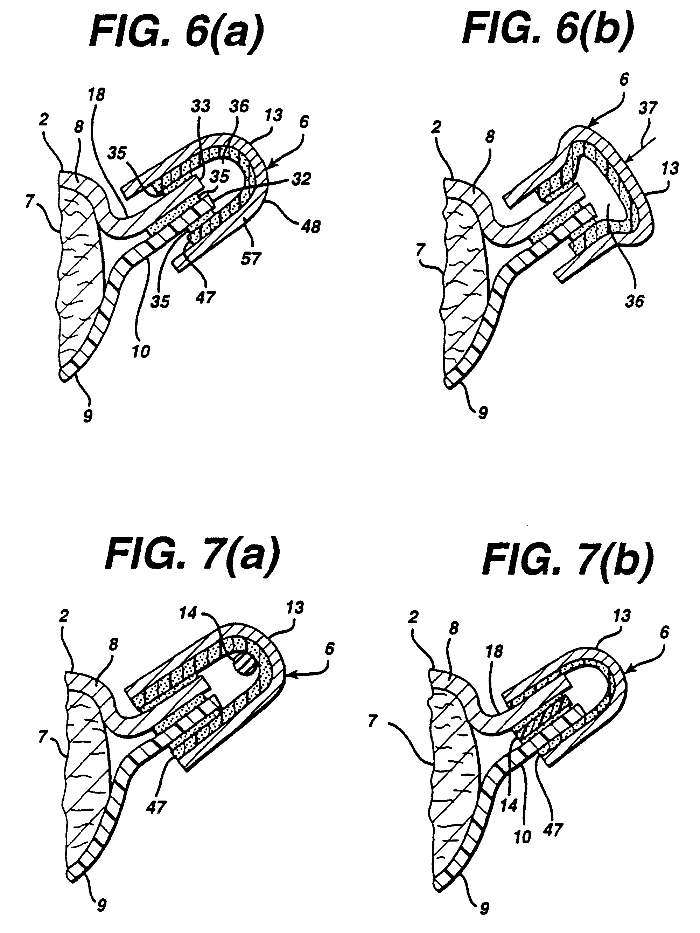

As shown in Figures 5 and 6(a), one embodiment of the invention comprises an absorbent article having side cuffs, wherein each compliant cuff 6 is formed by joining the portions 18 of the cover layer 8 adjacent its longitudinal edges 33 to the portions 10 of the barrier layer 9 adjacent its longitudinal edges 32 via an adhesive 35, thereby forming a flange, and enclosing the flange and the strip of high loft material 47 in a strip of an apertured polymeric film 57 attached thereto via adhesive 35. In the current embodiment, it is that the strip of resilient, fluid permeable, high loft material 47 cover at least the upper surface of the flange, and preferably covers both the upper, body-facing surface of the flange as well as the lower, garment facing surface of the flange. Covering both surfaces of the flange may be accomplished by adhering two separate strips of high loft material 47 to the outer surfaces of the flange, or by folding a single strip of high loft material around the outer edge margin of the flange, i.e. in a "C" fold configuration.

According to another embodiment of the current invention, the cuff 48 may be attached to the absorbent article, preferably to the flanges of the absorbent article, in a configuration so as to form a loop which encloses a cavity 36, discussed further below. The loop configuration of the cuff 6 provides considerable compliancy while the combination of high loft resilient material 48 with fluid repellant porous cover material provides a clean dry appearance to the absorbent article. Thus, the rigidity and directional stability provided by the resilient high loft material in the cuff 6 is not obtained at the detriment of comfort. Specifically, the distal ends 13 of the cuffs are readily deformed by an inward force 37, imparted by the user's thigh which act in the plane of the cuff, as shown in Figure 8. The force 37 is elastically absorbed by the flexure resistance of the high loft material in the compliant cuff which permits a bending deformation of the loop which flattens the cavity 36, as shown in Figure 6(b). In this manner the cuff 6 remains in sealing contact with the body throughout a range of motion due to the resilient, flexure resistant nature of the high loft material. Moreover, the compliancy of the cuff 6 can be varied by adjusting the size and shape of the cavity 36, and the thickness and resilience of the high loft material.

A further advantage of the flattening effect of the cuff 6 is that as the compression forces increase, the cuff geometry automatically adjusts itself so as to increase the area over which the contact force is distributed, thereby further minimizing the awareness of contact.

As shown in Figure 5, in the longitudinal middle section of the absorbent article, the cuffs 6 extend outward from the lateral sides of the absorbent article and are preferably maintained within the plane of the base portions of the cuff. That is it is preferred that the cuffs remain substantially flat, i.e. the cuffs preferably remain in a plane which is substantially parallel to the cover layer and/or the barrier layer of the absorbent article. However, the cuffs may optionally extend a distance above the body facing surface 16 of the central portion 2 -- specifically, above the portion of the body facing surface 16 that is adjacent the sides 30 of the central portion. In accordance with this aspect of the current invention, the distance by which the cuffs extend above the adjacent portion of the body facing surface 16 may be greater than zero to enhance a sealing contact with the user's body, as shown in Figure 8. However, this distance must not be so great, notwithstanding the aforementioned directional stability, that the cuffs 6 fold inwardly over the body facing surface 16 in use, thereby covering a substantial portion of the body facing surface and preventing it from passing fluid to the absorbent core 7.

As shown in Figure 8, the side edges 28 of the panty crotch 27 need not press directly against the cuffs to extend them into sealing contact with the perineum. As a result, the width D of the central portion 2 can be greater than the width C of the panty crotch 27, as shown in Figure 8, thereby permitting, if desired, the central portion to be sized to have maximum absorbency yet remain suitable for use with panties of any size.

In another embodiment, the absorbent article 1 is curved in the longitudinal direction so that it has an arcuate shape, thereby better conforming the shape of the absorbent article to that of the body and improving the fit. In accordance with this embodiment of the invention, elastic members may be incorporated into the loop type cuff 6 or alternatively the apertured polymeric film may be applied to the cuffs in tension to create an arcuate shape in the longitudinal direction of the absorbent article.

In one aspect of this embodiment, an elastic element 14, e.g., an elastic polyurethane foam, is laminated to the interior surface of the cuff 6, as shown in Figure 7(b). In this embodiment, the elastic element 47 extends essentially the length of the cuff 6 and may be attached to the cuff at its ends. The elastic element 14 may be tensioned or untensioned, where, in its untensioned state, the elastic element provides enhanced resilience to maintain the side cuffs ability to retain their original shape after the application of deformation forces from a user's thighs. Alternatively, the elastic foam may be applied to the absorbent article in tension to impart an arcuate shape to the absorbent article. In this embodiment, the elastic element, in its non-deformed state, is shorter than the cuff 6 so that the elastic element is placed in tension by extending it at least 15% when it is attached to the ends of the cuff. When released, the elastic element returns to its approximate original length, thereby forcing the article into an arcuate shape. In yet another alternative embodiment, the length of the elastic member may optionally span only a portion of the cuff length while still being placed in tension so as to impart an arcuate shape. In the preferred embodiment, the elastic element extends at least 30% of the length of the cuff.

The elastic element 14 may be disposed in the cavity 36 formed within the cuff 6 and attached thereto at its ends, as shown in Figure 7(a) or the elastic foam strip 14 may be disposed between the portions 18 and 10 of the cover layer and barrier layer that form the flange, as shown in Figure 7(b). Alternatively, the strip of high loft material 47 forming the cuff 6 could itself be a porous elastic foam applied to the absorbent article in tension, as shown in Figure 7(b).

The arcuate shape could also be imparted by applying to the cuffs heat shrinkable elements -- such as filaments formed from vinylidene chloride copolymer microtape, as disclosed in U.S. Patent No. 3,236,238 to Morse, hereby incorporated by reference in its entirety. Such filaments are heated after application to the cuffs, thereby causing them to shrink so as to impart an arcuate shape to the absorbent article.

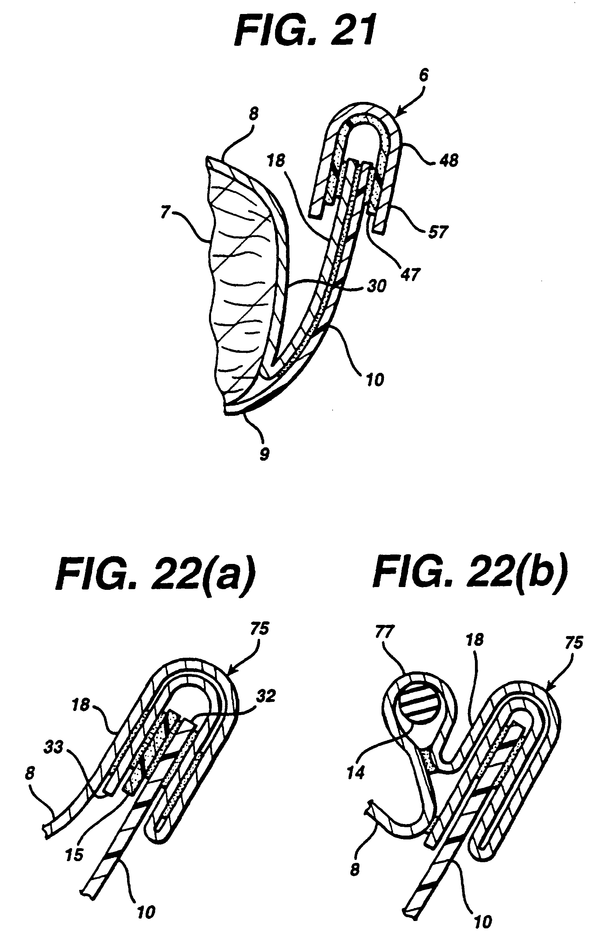

In Figures 5 and 6, the flange portion is shown as beginning near the top of the side 30 of the central portion 2. However, as shown in Figure 21, the flange could be formed by joining the portions 18 and 10 of the cover and base near the bottom of the side 30 and extending the flange upward therefrom to form the cuff.

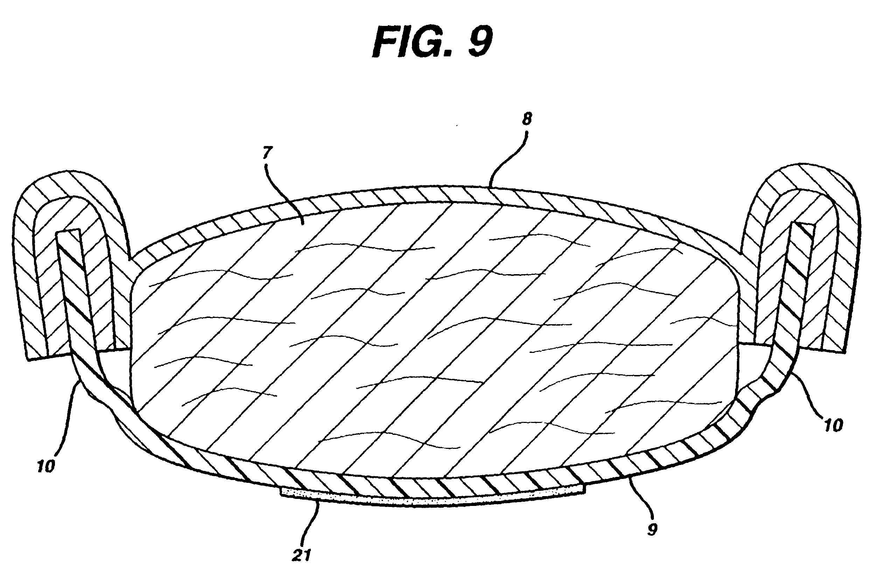

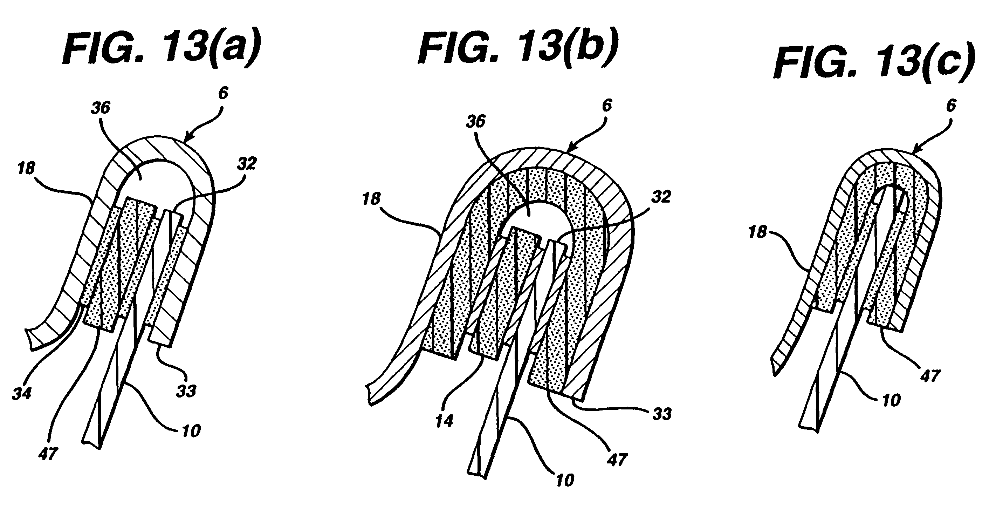

In yet another alternate embodiment of the absorbent article according to the current invention is shown in Figure 13 (b). According to this embodiment, the cuff further contains a substantially flat strip 14 of a flexible resilient material attached at its proximal end to the central portion 2. The strip 14 may be formed from a cross-linked foam, such as VOLARA™, supplied by Voltek, a division of Sekisui America Corporation of Lawrence, MA, having a thickness in the range of approximately 0.8 to 3mm (0.03 to 0.12 inch). The strip 14 is advantageously disposed between the portion 10 of the barrier layer 9 and the high loft material 47 adjacent their longitudinal edges 32 and 33, respectively, and attached via adhesive to each. Moreover, as shown in Figure 9, the barrier layer portion 10 may be extended so as to cover substantially all of the outward facing surface of the cuff, thereby further preventing leakage.

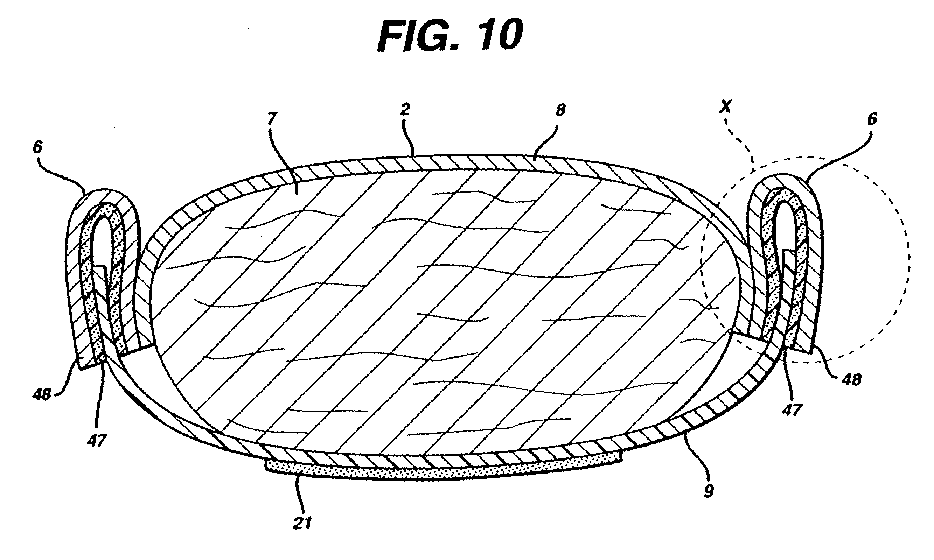

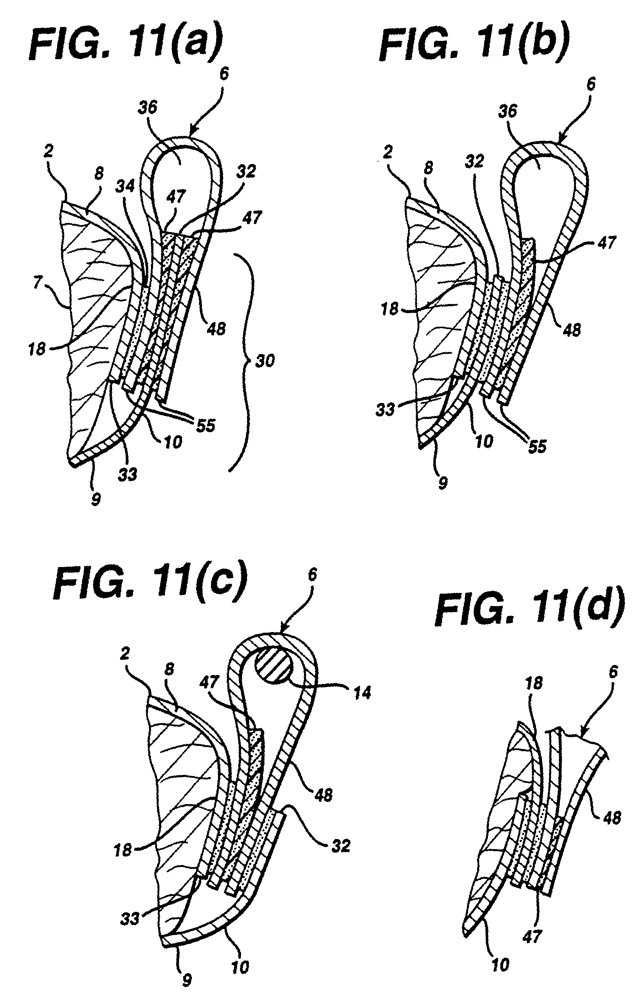

Figures 10 and 11 show another embodiment of the current invention. In this embodiment, the cuff 6 is formed by bringing together the longitudinal edges of a strip of resilient high loft material 47 covered with an apertured polymeric film so as to form a loop. As shown in Figure 11(a), the cuff 6 is attached to the sides 30 of the central portion 2 by joining, using adhesive 34, the interior surfaces of the strip 55 adjacent its longitudinal edges to the inward and outward facing surfaces of the portion 10 of the barrier layer 9 adjacent its longitudinal edge 32 so that the loop forms a cuff 6 that encloses the portion of the barrier layer adjacent its longitudinal edge. In addition, the outward facing surface of the portion 18 of the cover layer 8 that is adjacent its longitudinal edge 33 is joined to the inward facing surface of the cuff 6 by adhesive 34.

As shown in Figure 11(b), the cuff 6 could be formed by joining the interior surfaces of the two portions of the strip of material 55 adjacent its longitudinal edges directly together, and then attaching the inward facing surface of cuff 6 to the outward facing surface of the portion 10 of the barrier layer 9 adjacent its longitudinal edge 32 and attaching the inward facing surface of the portion of the barrier layer adjacent its longitudinal edge to the outward facing surface of the portion 18 of the cover layer 8 adjacent its longitudinal edge 33 so that the portion 10 of the barrier layer 9 is disposed between the cuff and the portion 18 of the cover layer 8. Also, as shown in Figure 11(c), the outward facing surface of the cuff 6 could be attached to the inward facing surface of the portion 10 of the barrier layer and the inward facing surface of the cuff attached to the outward facing surface of the portion 18 of the cover layer so that the cuff is disposed between the portions 10 and 18 of the barrier layer and cover layer, respectively.

Alternatively, as shown in Figure 11(d), the cuff 6 could be attached to the outward facing surface of the portion 18 of the cover layer 8 adjacent its longitudinal edge 33 and the inward facing surface of the portion of the cover layer adjacent its longitudinal edge attached to the outward facing surface of the portion 10 of the barrier layer 9 adjacent its longitudinal edge 32 so that the portion 18 of the cover layer 8 adjacent its longitudinal edge 33 is disposed between the cuff and the portion 10 of the barrier layer 9 adjacent its longitudinal edge 32.

Importantly, in each of the approaches to attaching the cuffs to the central portion 2 shown in Figures 10 and 11, the cuff 6 is attached to the lateral sides of the absorbent article 30 along a portion of the surfaces forming the sides of the cuff, rather than along its edges. Thus, at least a portion of each of the sides 30 of the central portion is formed from a laminate comprising layers of cuff, cover layer and barrier layer material. Unlike prior art attempts at forming cuffs, the cuffs are not attached along flexible joint lines adjacent the tops of the sides 30 of the central portion or transversely spaced apart from the sides 30, which would allow them to freely bend. Such prior art flexible joints have the undesirable characteristics of requiring the presence of elastic within the cuffs or contact between the elastic in the panty crotch and the cuffs in order to maintain them in the upright position. Such flexible joints also allow the cuffs to fold over the body facing surface 16 of the central portion, thereby reducing its effective area.

By contrast, the attachment method according to the embodiment of the current invention shown in Figures 10 and 11, gives adequate directional stability to the cuffs so that they will extend upward so as to make good sealing contact with the perineum without the incorporation of elastic members into the cuffs. Moreover, provided their width (as measured from the base portion to the distal end) is not excessive, the directional stability of the attachment method according to the current invention will prevent the cuffs from folding over the body facing surface 16 in use.

As shown in Figure 11, the loop type cuffs 6 optionally form cavities that impart compliancy to the cuff, as previously discussed with respect to the embodiment shown in Figure 6(a). Moreover, although, unlike some prior art cuffs, the cuffs according to the current invention do not require the presence of elastic members to cause them to extend upright, elastic members, such as those previously discussed with respect to the embodiment shown in Figures 6 and 7, may be advantageously incorporated into the loop type cuff 6 to create the arcuate shape. Figure 11(c) shows a cuff 41 in which an elastic filament 14 is disposed in the cavity 36.

Figure 12 shows still another embodiment of the current invention. In this embodiment, each cuff 6 is formed by extending the cover layer 8 so that the portion 18 adjacent its longitudinal edge 33 is wrapped around the portion 10 of the barrier layer adjacent its longitudinal edge 32, thereby forming a laminated cuff.

Figure 13 shows three embodiments of the cuffs 6 shown in Figure 12. As previously discussed, a cavity 36 can be formed inside the cuff so as to impart additional compliancy to the cuff 6. Moreover, a strip of elastic foam 14, placed in tension when applied to the absorbent article, can be disposed within the laminate to impart the aforementioned arcuate shape to the article. Additionally, a layer of porous, high loft foam 47 could be laminated to the inner surface of portion 18 of the cover layer 8 to further increase the compliancy of the cuff, as shown in the embodiment in Figure 13(b). Alternatively, the porous high loft foam 47 could be laminated to the outer surface of cover layer portion 18. As shown in Figure 13(c), a strip of elastic foam 47 placed in tension can be wrapped around the barrier layer portion 10 to provide both compliancy and shaping. In the embodiment shown in Figure 13(c), the cavity 36 has been eliminated, relying entirely on the foam strip 47 for compliancy.

Figure 22(a) shows another embodiment of the absorbent article shown in Figure 12, in which the cuff 75 is formed by folding the portion 18 of the cover layer over on itself before using it to enclose the portion 10 of the barrier layer, so that a double layer of the cover layer forms the cuff. As shown in Figure 22(b), an elastic element, attached to the absorbent article in tension, could be disposed within a secondary loop formed within the folded over portion of the cover layer portion 18 so as to form a secondary cuff 77 in addition to the primary cuff 75.

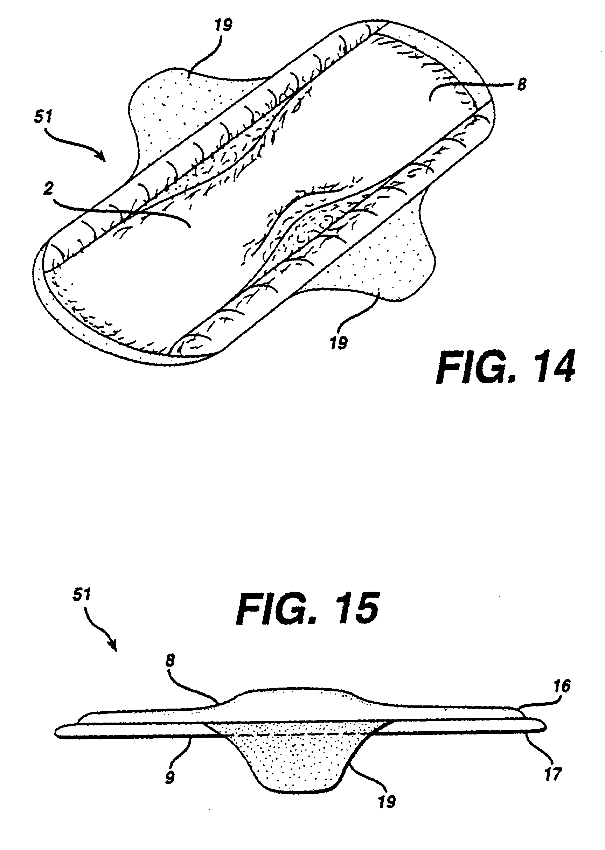

As shown in Figures 14 and 15, the current invention may be advantageously adapted for use in a winged absorbent article 51. The wings 19 extend laterally outward from the absorbent article central portion 2. Although preferably not including absorbent pulp materials, the wings 19 may include a body fluid impervious backing such as the materials described in connection with the above-mentioned body fluid impervious barrier layer 9. It is also expected that the wings 19 can comprise a body fluid pervious material, much like the above-mentioned body fluid pervious cover layer 8. According to the current invention, the wings 19 are of the "cut and paste" type -- that is, the wings are not integrally cut from the sheets of material forming the cover layer 8 and barrier layer 9 but are formed separately and attached to the central portion 2 via an adhesive. Such cut and paste wings are disclosed in U.S. Patent No. 4,900,320 (McCoy), hereby incorporated by reference in its entirety. Consequently, the wing material need not be of the type suitable for a pervious cover layer 8 or an impervious barrier layer 9.

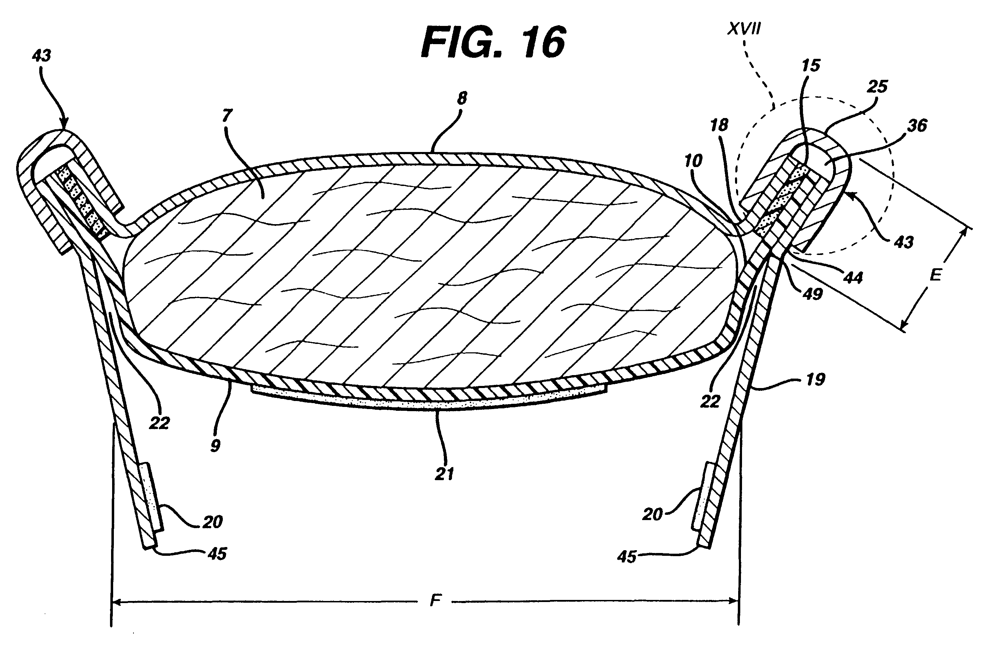

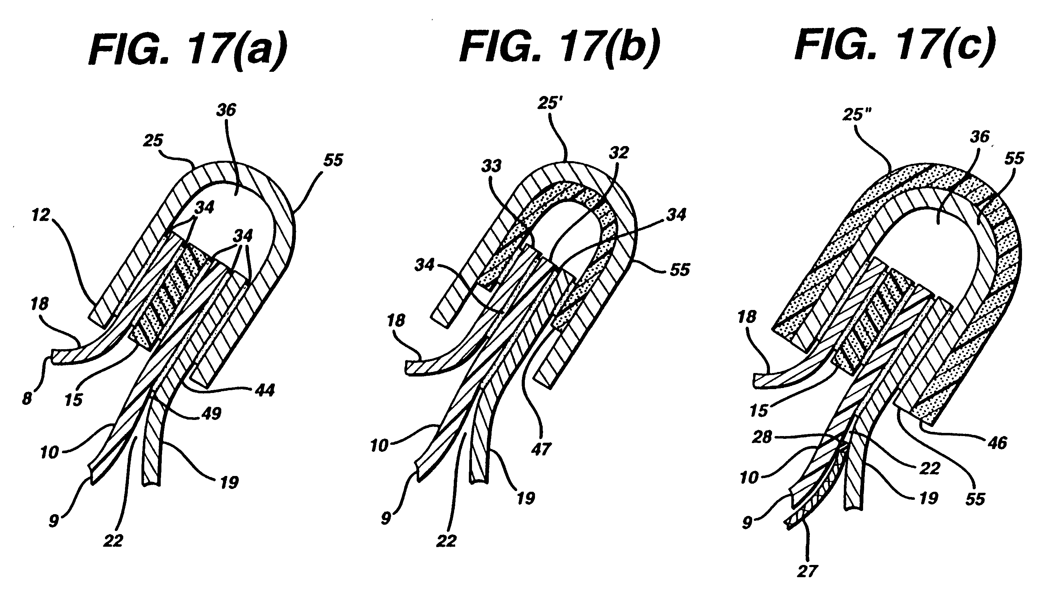

As shown in Figures 16 and 17, each cuff 43 may be formed by joining the portions 10 and 18 of the barrier layer 9 and cover layer 10, respectively, adjacent their longitudinal edges together via an adhesive 34, thereby forming a flange. A compliant cuff 25 is formed by enclosing the flange or joined portion in a strip of resilient high loft material 55, such as that used to form the cuffs 6 shown in Figures 6 and 7. The cuff 25 may be attached so as to form a loop that encloses a cavity 36, as previously discussed, thereby giving it considerable compliancy. As before, the size and shape of the cavity 36 can be adjusted to control the compliancy of the cuff.

A strip of elastic foam 15, placed in tension when applied to the absorbent article, may be disposed between the barrier layer and cover layer portions 10 and 18, respectively, that form the flange so as to impart the aforementioned arcuate shape to the absorbent article, as shown in Figure 17(a). Alternatively, as shown in Figure 17(b), a strip of elastic foam 47 may be laminated to the interior surface of the strip of material 55 that forms the cuff 25', as previously discussed with respect to the embodiment shown in Figure 6(a). As shown in Figure 17(c), a cuff 25" could be formed by wrapping a layer of fluid repellent porous foam material 46 around the strip of high loft material 55 to impart further cushioning for the cuff. The layer of foam 46 could itself be elasticized and applied to the absorbent article in tension, thereby eliminating the need for the elastic foam 15 to impart the arcuate shape.

Importantly, wings 19 are attached to the central portion 2 so that they cooperate with the cuffs 43 in use, as explained further below. In the preferred embodiment, the base 44 of each wing 19 is attached to a flange, as shown in Figure 17. Thus, as shown in Figure 17(b), a first strip of adhesive 34 is disposed between the portion 18 of cover layer 8 adjacent its longitudinal edge 33 and the portion 10 of the barrier layer 9 adjacent its longitudinal edge 33 and a second strip of adhesive is disposed between the opposite surface of the portion 10 of the barrier layer and the base 44 of the wing 19 so that the flange and wing base form a unitary structure. Alternatively, heat sealing could be used in place of adhesive strips 34. As a result this arrangement, the cuff 25' encloses the wing base 44, giving the absorbent article having cut and paste wings a more aesthetically pleasing appearance. More importantly, this method of attaching the wings to the absorbent article provides certain functional benefits, as described below.

The absorbent article 1 is generally applied to the crotch of a user's panty by placing the garment facing side of the absorbent article against the inside surface of the panty crotch 27, as shown in Figure 8. Pressure sensitive adhesive strips 21 are applied to the garment facing side of the absorbent article to help maintain the absorbent article in place. As used herein, the term "pressure-sensitive adhesive" refers to any releasable adhesive or releasable tenacious means. Adhesive compositions suitable for sanitary napkins, include, for example, water-based pressure-sensitive adhesives such as acrylate adhesives, rapid setting thermoplastic "hot melt", rubber adhesives, two-sided adhesive tape, and the like and combinations thereof.

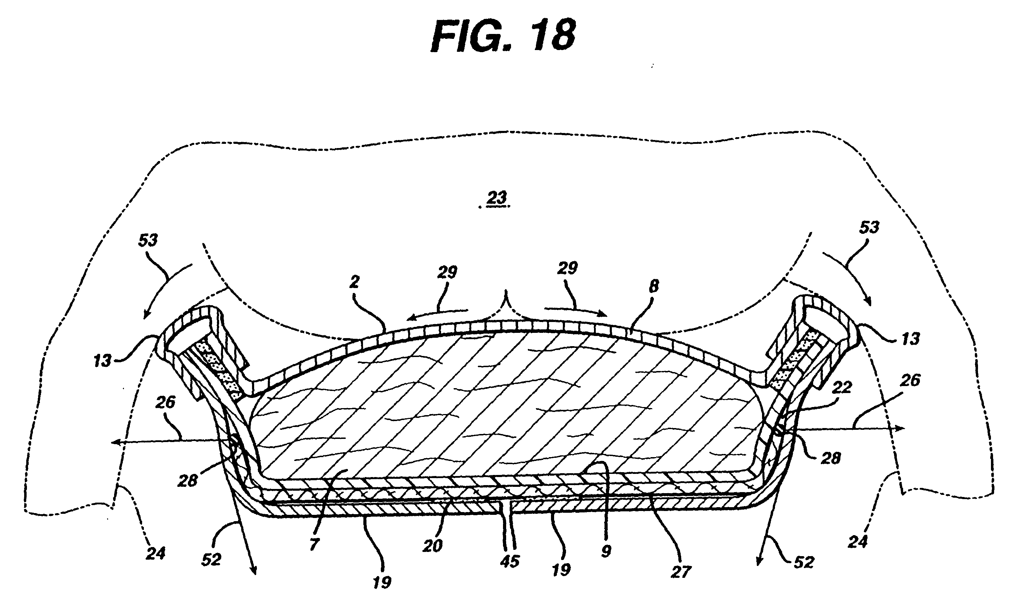

As is customary in the art, a paper release strip 56, which has been coated on one side, is applied to protect the adhesive strips 21 prior to use, as shown in Figures 4 and 5. The coating, which may be silicone, reduces the adherence to the adhesive of the coated side of the release strip. The release strip can be formed from any suitable sheet-like material which, when coated, adheres with sufficient tenacity to the adhesive to remain in place prior to use but which can be readily removed when the absorbent article is to be used. In use, the wings 19 are folded downward around the crotch 27 so that the edges of the wing tips 45 nearly abut each other and are secured to the underside of the crotch 27 via the adhesive 20, as shown in Figure 18. As is known in the art, the wings 19 serve to stabilize the absorbent article and protect the panty crotch 27 from side leakage. However, unlike the wings heretofore known in the art, when the user pulls the wings 19 according to the current invention around the edges of the panty crotch 27 and attaches them thereto by adhesive 20, downward forces 52 are applied to the cuffs through the wing bases 44. These downward forces 52 impose moments 53 that tend to rotate the cuffs downward, as shown in Figure 18. This downward rotation prevents the cuffs from folding inward over the body facing surface 16 of the cover layer 8, thereby ensuring that effective placement of the cuffs is maintained. As previously discussed, the folding of the cuffs over the body facing surface 16 reduces its effective area. Thus, according to the current invention, the wings 19 serve to place both the central portion 2 and the cuffs 43 into good contact with the body.

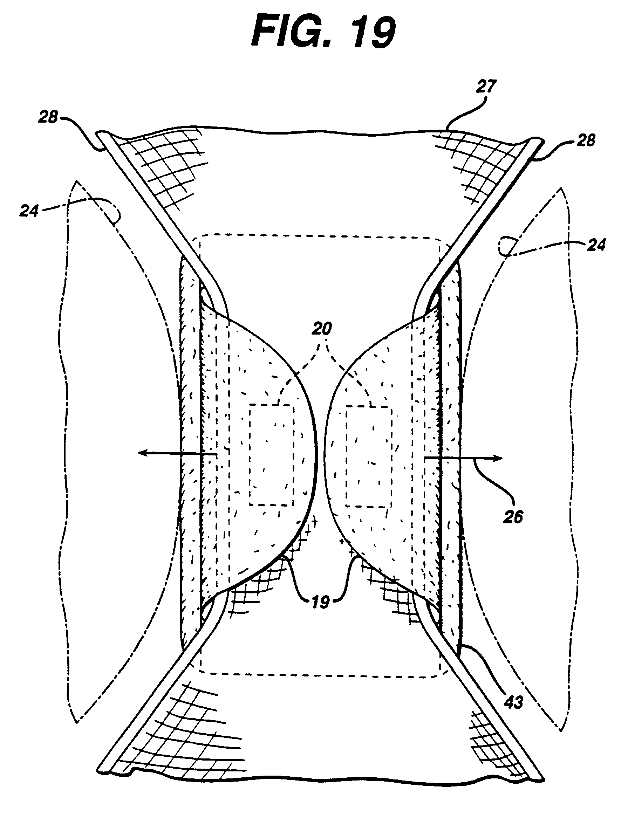

In the embodiment shown in Figure 16, the intersection 49 of the wing base 44 and the barrier layer 9 forms a pocket 22 disposed at, or slightly inward of, the proximal end of the cuff 43. As shown in Figures 17(c) and 18, the pockets 22 serve to contain the edge portions 28 of the panty crotch 27. According to the current invention, certain advantages are obtained by attaching the wings 19 so that the distance F, shown in Figure 16, between the pockets 22 is less than the width of the panty crotch 27 when the crotch is in its non-deformed state. Specifically, when the wings are attached to the panty crotch 27, as shown in Figures 18 and 19, the lateral compression of the crotch 27 causes the edges 28 to impart outward acting forces 26 on the wings 19. Since the wing base 44, barrier layer portion 10 and cover layer portion 18 are joined together so as to act in unison, the forces 26 are transmitted from the wings 19 to the cover layer 8, thereby placing the cover layer in tension, indicated by arrows 29 in Figure 18. This tension causes the cover layer 8 to be thrust upward so as to ensure good contact with the perineum 23. The tension also serves to prevent permanent deformation of the article due to lateral compression from the user's thighs since the panty crotch edges 28 act as a spring to restore a laterally compressed central portion 2 to its non-deformed shape.

Another advantage of the cuff/wing arrangement shown in Figures 16 and 17 is that the pockets 22, and therefore, the panty crotch edges 28, are disposed below the base 12 of the cuff 43. As a result, the cuffs 43 extend a distance E, shown in Figure 16, beyond the panty crotch edges. Unlike prior art attempts at sealing cuffs, the distance E is not limited to the thickness of the wing 19. Thus, the distal end 13 of the cuff makes sealing contact with the user's body regardless of the size or anatomical shape of the user or the panty crotch width.

The absorbent article shown in Figure 16 discloses another important aspect of the current invention. As is well known, the cover layer 8 can be formed from an apertured polymeric film, such as Reticulon™, APEX ™ available from Chicopee Mills, Inc. of New York, NY, or Dri-Weave™ by The Proctor & Gamble Company of Cincinnati, OH. Although such materials have the advantage of imparting a dry feeling, some of these materials are uncomfortable against the body, tending to produce a hot and sticky feeling, as previously discussed.

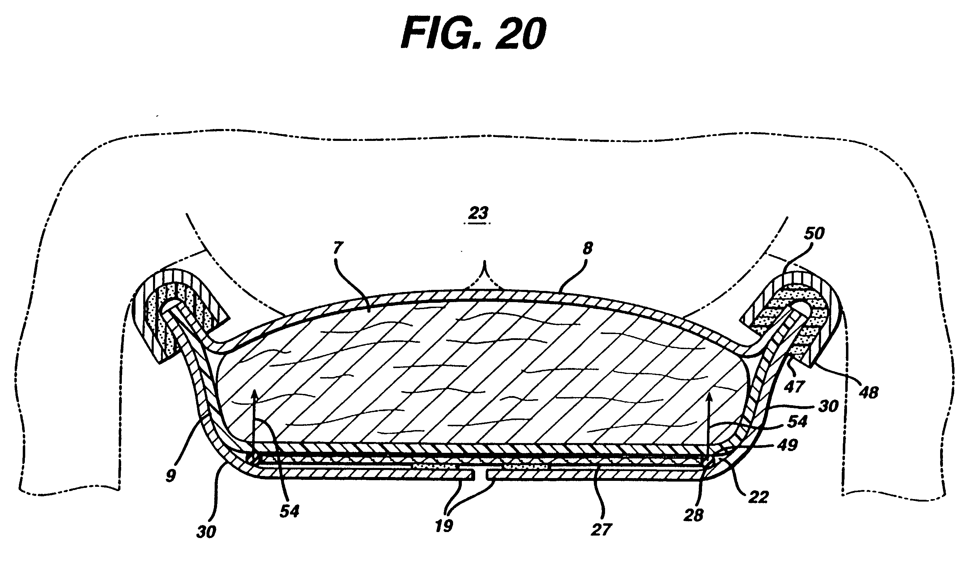

Consequently, as shown in Figure 16, according to the current invention, an apertured polymeric film can be used only for the portion of the cover layer 8 directly over the absorbent core 7, where it is most beneficial. A second, more comfortable material can be used for the cuff 25 that bears against the body under pressure. Preferably this second material is a fibrous woven or nonwoven material, which, as is well known in the art, has a comfortable feeling against the body. Alternatively, a cuff 50 could be formed from a laminate of a layer 48 of a fibrous woven or nonwoven material and a layer of a porous, high loft elastic foam 47, as shown in Figure 20.

According to the embodiment shown in Figure 20, the wings 19 are attached to the barrier layer 9 so that, unlike the embodiment shown in Figure 16, the initial intersection of the wings and the barrier layer occurs inward of the longitudinally extending sides 30 of the central portion 2, thereby similarly disposing the pockets 22. In this configuration, the elastic portions 28 of the panty crotch 27 impart forces 54 which act directly against the central portion 2 to enhance its contact with the perineum 23.

As the foregoing indicates, the method of the current invention affords great flexibility in the design of sanitary napkins, allowing the use of a wide range of cuff materials and allowing the cuffs to be attached to the absorbent article in various ways to achieve an optimum configuration. Moreover, although the invention has been explained with reference to a sanitary napkin, the invention is also suitable for use in other absorbent articles, such as incontinence pads and the like.

## Claims

1. An absorbent article (1) for use in a perineal area of a user's body to absorb body fluid, the absorbent article having right and left lateral sides and first and second transverse ends, the absorbent article comprising:
a) a fluid pervious first layer (8) forming a top body facing surface;
b) a fluid impervious second layer (9) forming a bottom garment facing surface opposite the body facing surface;
c) an absorbent core (7)positioned between the first layer and the second layer;
d) right and left cuffs (6) which extend along opposite right and left lateral sides of the absorbent article, the cuffs having a base portion and a distal end, each of the cuffs comprising a strip of a resilient, highloft, fluid permeable material (47) which is covered, at least in part, with a flexible, fluid repellent porous material (48), and wherein the right and left cuffs are attached along their respective base portions to the right and left lateral sides (30) of the absorbent article, respectively, such that the distal ends of the cuffs extend outward from the right and left lateral sides of the absorbent article.

2. The absorbent article (1) according to claim 1, wherein the fluid pervious first layer (8) has right and left approximately longitudinally extending edges, and the fluid impervious second layer (9) has right and left approximately longitudinally extending edges, and wherein the first layer (8) is joined to the second layer (9) adjacent to their respective right and left edges so as to form right and left flanges, wherein the right and left flanges are adjacent the right and left lateral sides of the absorbent article, and wherein the right and left longitudinally extending cuffs (6),are adhered to at least a portion of the right and left flanges, respectively.

3. The absorbent article (1) according to claim 1 or 2, wherein the longitudinal strip of resilient, fluid permeable, highloft material (47) comprises a material selected from the group consisting of polyester fibers, polyethylene/polyester bicomponent fibers, polyethylene/polypropylene bicomponent fibers, polypropylene/polyester bicomponent fibers, high melting/low melting polyester bicomponent fibers, air laid pulp, pulp-fiber blends, polymeric foam and combinations thereof.

4. The absorbent article (1) according to any one of claims 1 to 3, wherein the cuffs (6) further comprise a layer of a perforate or non-perforate flexible material which is laminated to the strip of resilient, fluid permeable, highloft material (47) to form a laminated structure, with the proviso that at least the top layer of the laminated structure comprises a fluid permeable material.

5. The absorbent article (1) according to claim 4, wherein the strip of resilient, fluid permeable, highloft material (47) and/or the layer of perforate or non-perforate flexible material is elastic and is applied to the cuff in tension, and wherein the tension is sufficient to form the absorbent article (1) into an arcuate shape in the longitudinal direction.

6. The absorbent article (1) according to any one of the preceding claims, wherein the fluid repellant porous material (48) is selected from the group consisting of apertured polymeric films, nonwoven fabrics and woven fabrics.

7. The absorbent article (1) according to claim 6, wherein the fluid repellant porous material (48) is a strip of apertured polymeric film and is applied to the absorbent article (1) in tension, wherein the tension is sufficient to form the absorbent article (1) into an arcuate shape in the longitudinal direction.

8. The absorbent article (1) according to any one of the preceding claims, wherein each of the cuffs (6) comprises a laminated structure having a top layer and a bottom layer, wherein one or more layers of polymeric film or polymeric foam material are laminated to one or more layers the resilient, fluid permeable, highloft material (47), with the proviso that the top layer of the laminated structure comprises a fluid permeable material.

9. The absorbent article (1) according to claim 8, wherein the resilient, fluid permeable, highloft material (47) is a fibrous woven or nonwoven material.

10. The absorbent article (1) according to claim 8 or 9, wherein at least one of the layers of polymeric film or polymeric foam material is tensioned prior to being laminated to the one or more layers of fibrous nonwoven material.

11. The absorbent article (1) according to any one of the preceding claims 1, wherein the right and left cuffs (6) further comprise first and second elastic members, respectively, wherein the elastic members are enclosed by strips of an apertured polymeric film, and wherein the first and second elastic members are disposed between and joined to the portions of the first and second layers adjacent their respective edges that form the flanges, the elastic members being in tension when applied to the article (1), and wherein the tension is sufficient to form the article (1) into an arcuate shape in the longitudinal direction,.

12. The absorbent article (1) according to any one of the preceding claims 6, wherein the strip forms a cavity (36) at the distal end of its respective cuff (6).

13. The absorbent article (1) according to claim 12, wherein the cavity forms a compliant portion at the distal end of the cuff (6) that deforms in response to compressive forces imparted by a user's body.

14. The absorbent article (1) according to claim 12 or 13, wherein each of the cuffs (6) further comprises a longitudinally extending elastic element disposed within its respective cavity (36), the elastic elements being in tension when applied to the article (1), wherein the tension is sufficient to form the article (1) into an arcuate shape in the longitudinal direction.

15. The absorbent article (1) according to any one of the preceding claims, further comprising right and left wings (19) each having a base portion and a distal end portion, the distal end portion being foldable over a crotch portion of a user's undergarment, the right and left wings (19) attached at their respective bases to portions of the right and left cuffs, respectively, and forming a part thereof.

16. The absorbent article (1) according to claim 15, wherein the bases of the right and left wings (19) are at least partially enclosed by the strips of material forming the right and left cuffs (6), respectively.

17. The absorbent article (1) according to any one of claims 1 to 14, further comprising right and left wings (19), the distal end portion being foldable over a crotch portion of a user's undergarment, the right and left wings attached at their respective bases to portions of the second layer (9) adjacent the right and left sides of the absorbent article (1), respectively.

18. The absorbent article(1) according to any one of claims 15 to 17, wherein the absorbent article (1) used in conjunction with an undergarment having a crotch portion, the crotch portion having right and left edges, and wherein the right and left wings (19) form right and left pockets which, when folded over the right and left edges of the crotch portion, respectively, restrain the right and left edges from contacting the cover layer of the absorbent article (1).

19. The absorbent article (1) according to claim 18, wherein the right and left cuffs (6) have base portions, and wherein the right and left pockets are disposed inwardly from the base portions of the right and left cuffs (6), respectively.

20. The absorbent article (1) according to any one of the preceding claims, wherein the absorbent core has an hourglass shape with enlarged end portions separated by a narrower center portion and wherein the center portion is thicker than the end portion.

21. The absorbent article (1) according to claim 20, wherein the center portion further comprises sphagnum moss.

22. An absorbent article (1) according to claim 1. and adapted for use in conjunction with an undergarment having a crotch portion having right and left edges, the absorbent article (1) comprising:
a) joining means for joining the first and second layers, thereby forming right and left flanges adjacent the right and left sides of the absorbent core (7), respectively; wherein each cuff (6) is attached along its respective base portion to the absorbent article adjacent the right and left flanges and wherein the distal end of each cuff (6) extends outward therefrom, whereby the distal ends of the cuffs (6) are compressed against a portion of the user's body in use;
b) right and left wings (19) each having a base portion and a distal end portion, the base portion being attached to the fluid impervious layer inwardly from the lateral edges of the absorbent article, thereby forming right and left pockets, the distal end portion adapted to fold over and retain the right and left edges of the undergarment crotch portion in the right and left pockets, respectively.

23. The absorbent article (1) according to claim 22, wherein the distance between the right and left pockets is less than the width of the undergarment crotch.

24. The absorbent article according to claim 22 or 23, wherein:
a) the first and second layers (8,9) each have first and second approximately longitudinally extending edges;
b) the first joining means comprises first adhesive disposed (i) between a portion of the first layer (8) adjacent its right edge and a portion of the second layer (9) adjacent its right edge and (ii) between a portion of the first layer (8) adjacent its left edge and a portion df the second layer (9) adjacent its left edge; and
c) the second joining means comprises second adhesive disposed (i) between the portion of the second layer (9) adjacent its right edge and the base portion of the right wing (9) and (ii) between the portion of the second layer (9) adjacent its left edge and the base portion of the left wing (19), the first and second adhesives disposed on opposite surfaces, respectively, of the portions of the second layer (9) adjacent its right an left edges.

25. The absorbent article (1) according to any one of claims 22 to 24, wherein the base portions of the right and left cuffs (6) are disposed outwardly from the right and left pockets, respectively.

26. The absorbent article (1) according to any one of claims 22 to 25, wherein the right and left sides of the absorbent core (7) are disposed outwardly from the right and left pockets, respectively, and wherein the right and left edges of the undergarment crotch portion impart upward acting forces directly to the central portion to maintain the absorbent article (1) in a body-contacting position during use.

27. The absorbent article (1) according to any one of claims 22 to 26, wherein the right and left cuffs (6) further comprise first and second strips of material enclosing at least a portion of the right and left flanges, respectively.

28. The absorbent article according to claim 27, wherein the base portions of the right and left wings (19) are attached to the second layer (9) at the right and left flanges, the bases forming a portion of the cuffs (6), and wherein the first and second strips enclose at least a portion of the bases of the right and left wings (19), respectively.

29. An absorbent article (1) according to claim 1, wherein: the first layer (8) has right and left approximately longitudinally extending edges, and the second layer (9) has right and left approximately longitudinally extending edges, and wherein the first layer (8) is joined to the second layer (9) adjacent to their respective right and left edges so as to form right and left flanges, wherein the right and left flanges are adjacent the right and left lateral sides of the absorbent article (1);
each of the cuffs (6) comprises a longitudinal strip of the resilient, fluid permeable, highloft material (47), and a longitudinal strip of an apertured polymeric film (48) which encloses the highloft material along at least a substantial portion of its length and wherein the right and left longitudinally extending cuffs (6) are adhered along their respective base portions to the right and left lateral sides (30) of the absorbent article (1); and
right and left wings, each having a base portion and a distal end portion, are attached at their respective base portions to the right and left flanges, respectively, and forming a part thereof, the distal ends being foldable over a crotch portion of a user's undergarment.

30. The absorbent article (1) according to claim 29, wherein the absorbent core (7) has an hourglass shape with enlarged end portions separated by a narrower center portion and wherein the center portion is thicker than the end portion.

31. The absorbent article according to claim 30, wherein the center portion further comprises sphagnum moss.

## Patentansprüche

1. Absorptionsfähiges Produkt (1) zur Verwendung im perinealen Bereich des Körpers eines Benutzers zur Absorption von Körperflüssigkeit, wobei das absorptionsfähige Produkt rechte und linke laterale Seiten und erste und zweite querverlaufende Enden aufweist, wobei das absorptionsfähige Produkt umfaßt:
a) eine flüssigkeitsdurchlässige erste Schicht (8), die eine obere, dem Körper zugewandte Oberfläche bildet;
b) eine flüssigkeitsundurchlässige zweite Schicht (9), die gegenüber der dem Körper zugewandten Fläche eine untere, der Kleidung zugewandte Fläche bildet;
c) einen absorptionsfähigen Kern (7), der zwischen der ersten und der zweiten Schicht angeordnet ist;
d) rechte und linke Aufschläge (6), die sich entlang der gegenüberliegenden rechten und linken lateralen Seiten des absorptionsfähigen Produkts erstrecken, wobei die Aufschläge ein Basisteil und ein distales Ende aufweisen, jeder der Aufschläge einen Streifen aus einem biegsamen, hochgradig speicherfähigen, flüssigkeitsdurchlässigen Material (47) umfaßt, das zumindest teilweise mit einem flexiblen, flüssigkeitsabweisenden porigen Material (48) bedeckt ist, und wobei die rechten und linken Aufschläge entlang ihrer entsprechenden Basisbereiche jeweils an den rechten und linken lateralen Seiten (30) des absorptionsfähigen Produkts in einer Weise befestigt sind, daß sich die distalen Enden der Aufschläge von den rechten und linken lateralen Seiten des absorptionsfähigen Produkts nach außen erstrecken.

2. Absorptionsfähiges Produkt (1) nach Anspruch 1, wobei die flüssigkeitsdurchlässige erste Schicht (8) rechte und linke, sich annähernd längsseitig erstreckende Ränder aufweist, und die flüssigkeitsundurchlässige zweite Schicht (9) rechte und linke, sich annähernd längsseitig erstreckende Ränder aufweist, und wobei die erste Schicht (8) mit der zweiten Schicht (9), an deren entsprechenden rechten und linken Rändern verbunden ist, damit sich rechte und linke Flansche bilden, wobei die rechten und linken Flansche an die rechten und linken lateralen Seiten des absorptionsfähigen Produkts angrenzen, und wobei die sich längsseitig erstreckenden rechten und linken Aufschläge (6) an mindestens einem Teil des jeweils rechten und linken Flanschs angeheftet sind.

3. Absorptionsfähiges Produkt (1) nach Anspruch 1 oder 2, wobei der Längsstreifen aus biegsamem, flüssigkeitsdurchlässigem, hochgradig speicherfähigem Material (47) ein Material umfaßt, das aus der Gruppe ausgewählt ist, bestehend aus Polyesterfasern, Polyethylen-/Polyester-Zweikomponentenfasern, Polyethylen-/Polypropylen-Zweikomponentenfasern, Polypropylen-/Polyester-Zweikomponentenfasern, hochschmelzendes/ niedrigschmelzendes Polyester-Zweikomponentenfasern, unter Luftstrom aufgebrachtem Weichstoff, Mischungen von Weichstoff-Fasern, polymerem Schaum und Kombinationen daraus.

4. Absorptionsfähiges Produkt (1) nach einem der Ansprüche 1 bis 3, wobei die Aufschläge (6) weiterhin eine Schicht aus einem perforierten oder nicht-perforierten flexiblen Material umfassen, das auf den Streifen aus biegsamem, flüssigkeitsdurchlässigem, hochgradig speicherfähigem Material (47) aufgeschichtet ist, um eine geschichtete Struktur zu bilden, unter der Voraussetzung, daß zumindest die obere Schicht der geschichteten Struktur ein Flüssigkeitsdurchlässiges Material umfaßt.

5. Absorptionsfähiges Produkt (1) nach Anspruch 4, wobei der Streifen aus biegsamem, flüssigkeitsdurchlässigem, hochgradig speicherfähigem Material (47) und/oder die Schicht aus perforiertem oder nicht-perforiertem flexiblem Material elastisch ist und an den Aufschlag unter Spannung angebracht ist, und wobei die Spannung ausreicht, um dem absorptionsfähigen Produkt in Längsrichtung eine bogenartige Form zu geben.

6. Absorptionsfähiges Produkt nach einem der voranstehenden Ansprüche, wobei das flüssigkeitsabweisende porige Material (48) ausgewählt ist aus der Gruppe bestehend aus, mit Öffnungen versehenen polymeren Filmen, ungewebten Stoffen und gewebten Stoffen.

7. Absorptionsfähiges Produkt (1) nach Anspruch 6, wobei das flüssigkeitsabweisende porige Material (48) ein Streifen aus mit Öffnungen versehenem polymerem Film ist und an das absorptionsfähige Produkt (1) unter Spannung angebracht ist, wobei die Spannung ausreicht, um dem absorptionsfähigen Produkt (1) in Längsrichtung eine bogenförmige Form zu geben.

8. Absorptionsfähiges Produkt (1) nach einem der voranstehenden Ansprüche, wobei jeder der Aufschläge (6) eine geschichtete Struktur mit einer oberen Schicht und einer unteren Schicht umfaßt, wobei eine oder mehrere Schichten aus polymerem Film oder polymerem Schaummaterial auf eine oder mehrere Schichten des biegsamen, flüssigkeitsdurchlässigen, hochgradig speicherfähigen Materials (47) aufgeschichtet sind, unter der Voraussetzung, daß die obere Schicht der geschichteten Struktur ein flüssigkeitsdurchlässiges Material umfaßt.

9. Absorptionsfähiges Produkt (1) nach Anspruch 8, wobei das biegsame, flüssigkeitsdurchlässige, hochgradig speicherfähige Material (47) ein gewebtes oder nicht-gewebtes faseriges Material ist.

10. Absorptionsfähiges Produkt (1) nach Anspruch 8 oder 9, wobei mindestens eine der Schichten aus polymerem Film oder polymerem Schaummaterial gespannt wird, bevor sie auf die eine oder mehrere Schichten aus nicht-gewebtem faserigem Material geschichtet wird.

11. Absorptionsfähiges Produkt (1) nach einem der voranstehenden Ansprüche, wobei die rechten und linken Aufschläge (6) weiterhin jeweils erste und zweite elastische Glieder umfassen, wobei die elastischen Glieder von Streifen eines mit Öffnungen versehenen polymeren Films eingeschloßessen sind, und wobei die ersten und zweiten elastischen Glieder zwischen den Teilen der ersten und zweiten Schichten angeordnet und mit diesen verbunden sind, angrenzend an deren jeweiligen Ränder, die die Flansche bilden, wobei die elastischen Glieder unter Spannung sind, wenn sie an dem Produkt (1) angebracht werden, und wobei die Spannung ausreicht, um dem Produkt (1) in Längsrichtung eine bogenartige Form zu geben.

12. Absorptionsfähiges Produkt (1) nach einem der voranstehenden Ansprüche, wobei der Streifen am distalen Ende seines entsprechenden Aufschlags (6) einen Hohlraum (36) bildet.

13. Absorptionsfähiges Produkt (1) nach Anspruch 12, wobei der Hohlraum am distalen Ende des Aufschlags (6) einen nachgiebigen Teil bildet, der sich unter zusammendrückend wirkenden Kräften, die vom Körper eines Verwenders ausgehen, verformt.

14. Absorptionsfähiges Produkt (1) nach Anspruch 12 oder 13, wobei jeder der Aufschläge (6) weiterhin ein sich in Längsrichtung erstreckendes elastisches Element umfaßt, das in seinem entsprechenden Hohlraum (36) angebracht ist, wobei die elastischen Elemente unter Spannung sind, wenn sie am Produkt (1) angebracht werden, wobei die Spannung ausreicht, um dem Produkt (1) in Längsrichtung eine bogenartige Form zu geben.

15. Absorptionsfähiges Produkt (1) nach einem der voranstehenden Ansprüche, das weiterhin rechte und linke Flügel (19) umfaßt, wobei jeder ein Basisteil und einen distalen Endbereich aufweist, wobei der distale Endbereich über einen Schrittbereich der Unterwäsche eines Verwenders faltbar ist, und die rechten und linken Flügel (19) mit ihrer jeweiligen Basis an Teilen der jeweils rechten und linken Aufschläge angebracht sind und einen Teil davon bilden.

16. Absorptionsfähiges Produkt (1) nach Anspruch 15, wobei die Basen der rechten und linken Flügel (19) mindestens teilweise von den Streifen aus Material eingeschlossen sind, das die jeweils rechten und linken Aufschläge (6) bildet.

17. Absorptionsfähiges Produkt (1) nach einem der Ansprüche 1 bis 14, das weiterhin rechte und linke Flügel (19) umfaßt, wobei der distale Endbereich über einen Schrittbereich der Unterwäsche eines Benutzers faltbar ist, und die rechten und linken Flügel an ihrer jeweiligen Basis an Teilen der zweiten Schicht (9), die an die jeweils rechten und linken Seiten des absorptionsfähigen Produkts (1) grenzen, befestigt sind.

18. Absorptionsfähiges Produkt (1) nach einem der Ansprüche 15 bis 17, wobei das absorptionsfähige Produkt (1), das in Verbindung mit Unterwäsche mit Schrittbereich, wobei der Schrittbereich linke und rechte Ränder aufweist, verwendet wird, und wobei die rechten und linken Flügel (19) rechte und linke Taschen bilden, die, wenn jeweils über die rechten bzw. linken Ränder des Schrittbereichs gefaltet, die rechten und linken Ränder daran hindern, mit der Deckschicht des absorptionsfähigen Produkts (1) in Kontakt zu kommen.

19. Absorptionsfähiges Produkt (1) nach Anspruch 18, wobei die rechten und linken Aufschläge (6) Basisteile aufweisen, und wobei die rechten und linken Taschen einwärts von den Basisteilen der jeweils rechten bzw. linken Aufschläge (6) angeordnet sind.

20. Absorptionsfähiges Produkt (1) nach einem der voranstehenden Ansprüche, wobei der absorptionsfähige Kern die Form eines Uhrglases mit verlängerten Endteilen, abgeteilt durch einen schmalen zentralen Teil, hat, und wobei der zentrale Teil dicker ist als das Endteil.

21. Absorptionsfähiges Produkt (1) nach Anspruch 20, wobei der zentrale Teil weiterhin Sphagnum umfaßt.

22. Absorptionsfähiges Produkt (1) nach Anspruch 1 und geeignet für den Gebrauch in Verbindung mit einer Unterwäsche, die einen Schrittbereich mit rechten und linken Rändern aufweist, wobei das absorptionsfähige Produkt (1) umfaßt:
(a) Verbindungsmittel zur Verbindung der ersten und zweiten Schichten, wodurch rechte und linke Flansche gebildet werden, die jeweils an die rechten und linken Seiten des absorptionsfähigen Kerns (7) angrenzen;
wobei jeder Aufschlag (6) entlang seines jeweiligen Basisteils an dem absorptionsfähigen Produkt befestigt ist, angrenzend an die rechten und linken Flansche, und wobei sich die distalen Enden jedes Aufschlags (6) von dort nach außen erstrecken, wodurch die distalen Enden der Aufschläge (6) während des Gebrauchs gegen einen Teil des Körpers des Verwenders gedrückt werden;
(b) rechte und linke Flügel (19), jeder mit einem Basisteil und einem distalen Endteil, wobei das Basisteil an der flüssigkeitsundurchlässigen Schicht einwärts von den seitlichen Rändern des absorptionsfähigen Produkts befestigt ist, wodurch rechte und linke Taschen gebildet werden, und das distale Endteil dazu geeignet ist, sich umzufalten und die rechten und linken Ränder des Schrittbereichs der Unterwäsche in den jeweils rechten und linken Taschen zu halten.

23. Absorptionsfähiges Produkt (1) nach Anspruch 22, wobei der Abstand zwischen den rechten und linken Taschen geringer ist, als die Breite des Unterwäsche-Schritts.

24. Absorptionsfähiges Produkt nach Anspruch 22 oder 23, wobei:
(a) die ersten und zweiten Schichten (8,9) jeweils erste und zweite sich in Längsrichtung erstreckende Ränder aufweisen;
(b) die ersten Verbindungsmittel ein erstes Klebemittel umfassen, das (i) zwischen einem Teil der ersten Schicht (8), das angrenzend an ihren rechten Rand liegt, und einen Teil der zweiten Schicht (9), der angrenzend an deren rechten Rand liegt, angeordnet ist; und (ii) zwischen einem Teil der ersten Schicht (8), der angrenzend an ihren linken Rand liegt, und einem Teil der zweiten Schicht (9), der angrenzend an deren linken Rand liegt, angeordnet ist; und
(c) die zweiten Verbindungmittel ein zweites Klebemittel umfassen, das (i) zwischen dem Teil der zweiten Schicht (9), der angrenzend an ihrem rechten Rand und dem Basisteil des rechten Flügels (9) liegt, und (ii) zwischen dem Teil der zweiten Schicht (9), der angrenzend an deren linken Rand und dem Basisteil des linken Flügels (19) liegt, angeordnet ist, und die ersten und zweiten Klebemittel auf jeweils einander gegenüberliegenden Flächen der Teile der zweiten Schicht (19), die neben deren rechten und linken Rändern liegen, angeordnet sind.

25. Absorptionsfähiges Produkt (1) nach einem der Ansprüche 22 bis 24, wobei die Basisteile der rechten und linken Aufschläge (6) auswärts jeweils der rechten und linken Taschen angeordnet sind.

26. Absorptionsfähiges Produkt (1) nach einem der Ansprüche 22 bis 25, wobei die rechten und linken Seiten des absorptionsfähigen Kerns (7) auswärts von jeweils den rechten und linken Taschen angeordnet sind, und wobei die rechten und linken Ränder des Unterwäsche-Schrittbereichs aufwärts gerichtete Kräfte direkt auf den zentralen Teil bewirken, um das absorptionsfähige Produkt (1) während des Gebrauchs in einer körpernahen Position zu halten.

27. Absorptionsfähiges Produkt (1) nach einem der Ansprüche 22 bis 26, wobei die rechten und linken Aufschläge (6) weiterhin erste und zweite Streifen von Material umfassen, die mindestens einen Teil der jeweils rechten und linken Flansche einschliessen.

28. Absorptionsfähiges Produkt nach Anspruch 27, wobei die Basisteile der rechten und linken Flügel (19) an der zweiten Schicht (9) an den rechten und linken Flanschen befestigt sind, wobei die Basen einen Teil der Aufschläge (6) bilden, und wobei die ersten und zweiten Streifen mindestens einen Teil der Basen der jeweils rechten und linken Flügel (19) einschließen.

29. Absorptionsfähiges Produkt (1) nach Anspruch 1, wobei:
die erste Schicht (8) rechte und linke, sich annähernd der Länge nach erstreckende Ränder aufweist, und die zweite Schicht (9) rechte und linke, sich annähernd der Länge nach erstreckende Ränder aufweist, und wobei die erste Schicht (8) mit der zweiten Schicht (9) dort verbunden ist, wo ihre jeweiligen rechten und linken Ränder aneinandergrenzen, um so rechte und linke Flansche zu bilden, wobei die rechten und linken Flansche neben den rechten und linken lateralen Seiten des absorptionsfähigen Produkts (1) liegen;
jeder der Aufschläge (6) einen Längsstreifen aus dem biegsamen, flüssigkeitsdurchlässigen, hochgradig speicherfähigen Material (47) umfaßt, und einen Längsstreifen aus einem, mit Öffnungen versehenen polymeren Film (48), der das hochgradig speicherfähige Material entlang mindestens eines wesentlichen Teils seiner Länge einschließt, und wobei die rechten und linken sich der Länge nach erstreckenden Aufschläge (6) entlang ihrer jeweiligen Basisteile an die rechten und linken lateralen Seiten (30) des absorptionsfähigen Produkts (1) angeheftet sind; und
rechte und linke Flügel, jeder mit einem Basisteil und einem distalen Endteil, die an ihren jeweiligen Basisteilen an den jeweiligen rechten und linken Flanschen befestigt sind und
einen Teil davon bilden, wobei die distalen Enden über den Schrittbereich der Unterwäsche eines Verwenders faltbar sind.

30. Absorptionsfähiges Produkt (1) nach Anspruch 29, wobei der absorptionsfähige Kern (7) die Gestalt eines Uhrglases mit verlängerten Endteilen, abgeteilt durch einen schmaleren zentralen Teil aufweist, und wobei der zentrale Teil dicker ist als der Endteil.

31. Absorptionsfähiges Produkt (1) nach Anspruch 30, wobei der zentrale Teil weiterhin Sphagnum umfaßt.

## Revendications

1. Article absorbant pour emploi dans la région périnéale du corps d'un utilisateur pour absorber les fluides du corps, ledit article absorbant présentant des côtés latéraux à droite et à gauche et une première et une seconde extrémités transversales, ledit article absorbant comportant :
a) une première couche (8) imperméable aux fluides formant une surface qui fait face au corps ;
b) une seconde couche (9) imperméable aux fluides formant une surface qui fait face au vêtement opposée à la surface qui fait face au corps ;
c) un noyau absorbant (7) positionné entre la première couche et la seconde couche ;
d) des manchettes (6) s'étendant longitudinalement à droite et à gauche, les manchettes présentant une portion de base et une extrémité distale, chacune des manchettes comportant une bande longitudinale d'un matériau élastique, densité basse, et épais , (47), perméable aux fluides, qui est recouvert, au moins en partie, par un matériau poreux (48) repoussant les fluides, et dans lequel les manchettes qui s'étendent longitudinalement à droite et à gauche sont attachées, le long de leurs portions de base respectives, aux côtés latéraux de droite et de gauche (30) de l'article absorbant, respectivement, de sorte que les extrémités distales des manchettes s'étendent à l'extérieur des bords latéraux de droite et de gauche de l'article absorbant.

2. L'article absorbant (1) selon la revendication 1, dans lequel la première couche (8), perméable aux fluides, présente des bords s'étendant approximativement longitudinalement à droite et à gauche, et la seconde couche (9), imperméable aux fluides, présente des bords s'étendant approximativement longitudinalement à droite et à gauche, et dans lequel la première couche (8) est jointe à la seconde couche (9), près de leurs bords, de droite et de gauche, respectifs, de façon à former des bordures de droite et de gauche, dans lequel les bordures de droite et de gauche sont adjacentes aux côtés latéraux de droite et de gauche de l'article absorbant et dans lequel des manchettes (6), s'étendant longitudinalement à droite et à gauche, sont attachées à au moins une portion des bordures de droite et de gauche, respectivement.

3. L'article absorbant (1) selon la revendication 1 ou 2, dans lequel la bande longitudinale de matériau (47) à déformation non permanente, perméable aux fluides, densité basse, et épais , comporte un matériau choisi dans le groupe constitué de fibres de polyester, fibres à deux composants polyéthylène/polyester, fibres à deux composants polyéthylène/polypropylène, fibres à deux composants polypropylène/polyester, fibres à deux composants polyester fondant à haute température/polyester fondant à basse température, pulpe exposée à l'air, mélange pulpe-fibres, mousse polymère et leurs combinaisons.

4. L'article absorbant (1) selon l'une quelconque des revendications 1 à 3, dans lequel les manchettes (6) comportent en outre une couche d'un matériau flexible, perforé ou non perforé, qui est stratifié sur la bande de matériau (47) à déformation non permanente, perméable aux fluides, densité basse, et épais , pour former une structure stratifiée, en stipulant qu'au moins la couche supérieure de la structure stratifiée comporte un matériau perméable aux fluides.

5. L'article absorbant (1) selon la revendication 4, dans lequel la bande de matériau (47) à déformation non permanente, perméable aux fluides, densité basse, et épais , et/ou la couche de matériau flexible, perforé ou non perforé, sont élastiques et appliquées sur la manchette sous contrainte de traction, et dans lequel la contrainte de traction est suffisante pour donner à l'article absorbant (1) une forme en arc selon la direction longitudinale.

6. L'article absorbant (1) selon l'une quelconque des revendications précédentes, dans lequel le matériau poreux (48), repoussant les fluides, est choisi dans le groupe constitué de films polymères perforés, de tissus non tissés et de tissus tissés.

7. L'article absorbant (1) selon la revendication 6, dans lequel le matériau poreux (48) repoussant les fluides, est une bande de film polymère perforé et est appliqué à l'article absorbant (1) sous contrainte de traction, dans lequel la contrainte de traction est suffisante pour donner à l'article absorbant (1) une forme en arc selon la direction longitudinale.

8. L'article absorbant (1) selon l'une quelconque des revendications précédentes, dans lequel chacune des manchettes (6) comporte une structure stratifiée présentant une couche supérieure et une couche inférieure, dans lequel une couche, ou plusieurs couches, de film polymère ou de matériau mousse polymère sont stratifiées sur une ou plusieurs couches du matériau (47) à déformation non permanente, perméable aux fluides, densité basse, et épais , en stipulant que la couche supérieure de la structure stratifiée comporte un matériau perméable aux fluides.

9. L'article absorbant (1) selon la revendication 8, dans lequel le matériau (47) à déformation non permanente, perméable aux fluides, densité basse, et épais , est un matériau fibreux tissé ou non tissé.

10. L'article absorbant (1) selon la revendication 8 ou 9, dans lequel au moins l'une des couches de film polymère ou de matériau mousse polymère est mise sous contrainte de traction avant d'être stratifiée sur une ou plusieurs couches de matériau fibreux non tissé.

11. L'article absorbant (1) selon l'une quelconque des revendications précédentes, dans lequel les manchettes (6) de droite et de gauche comportent en outre un premier et un second éléments élastiques, respectivement, dans lequel les éléments élastiques sont enclos dans des bandes de film polymère perforé, et dans lequel le premier et le second éléments élastiques sont disposés entre les, et joints aux, portions de la première et de la seconde couches près de leurs bords respectifs qui forment les bordures, les éléments élastiques étant sous contrainte de traction lorsqu'on les applique à l'article (1), et dans lequel la contrainte de traction est suffisante pour donner à l'article (1) une forme en arc selon la direction longitudinale.

12. L'article absorbant (1) selon l'une quelconque des revendications précédentes, dans lequel la bande forme une cavité (36) à l'extrémité distale de sa manchette respective (6).

13. L'article absorbant (1) selon la revendication 12 dans lequel la cavité forme à l'extrémité distale de la manchette (6) une portion qui peut se conformer et qui se déforme en réponse à des forces de compression exercées par le corps d'un utilisateur.

14. L'article absorbant (1) selon la revendication 12 ou 13, dans lequel chacune des manchettes (6) comporte en outre un élément élastique, s'étendant longitudinalement, disposé à l'intérieur de sa cavité respective (36), les éléments élastiques étant sous contrainte de traction lorsqu'on les applique à l'article (1), la contrainte de traction étant suffisante pour donner à l'article (1) une forme en arc selon la direction longitudinale.

15. L'article absorbant (1) selon l'une quelconque des revendications précédentes, comportant en outre des ailes (19) de droite et de gauche, chacune présentant une portion de base et une portion d'extrémité distale, la portion d'extrémité distale pouvant se replier par-dessus une portion formant entrejambe du sous-vêtement d'un utilisateur, les ailes (19) de droite et de gauche étant attachées, par leur base respective, à des portions des manchettes de droite et de gauche, respectivement, et en faisant partie.

16. L'article absorbant (1) selon la revendication 15, dans lequel les bases des ailes (19) de droite et de gauche sont au moins partiellement encloses dans les bandes de matériau formant les manchettes (6) de droite et de gauche, respectivement.

17. L'article absorbant (1) selon l'une quelconque des revendications 1 à 14, comportant en outre des ailes (19) de droite et de gauche, la portion d'extrémité distale pouvant se replier par-dessus une portion formant entrejambe du sous-vêtement d'un utilisateur, les ailes de droite et de gauche étant attachées, par leur base respective, aux portions de la seconde couche (9) près des côtés de droite et de gauche de l'article absorbant (1), respectivement.

18. L'article absorbant (1) selon l'une quelconque des revendications 15 à 17, dans lequel l'article absorbant (1) est utilisé en conjonction avec un sous-vêtement présentant une portion d'entrejambe, la portion d'entrejambe ayant des bords de droite et de gauche, et dans lequel les ailes (19), de droite et de gauche, forment des poches de droite et de gauche qui, lorsqu'elles sont repliées par-dessus les bords de droite et de gauche de la portion d'entrejambe, respectivement, empêchent les bords de droite et de gauche de venir en contact avec la couche supérieure de l'article absorbant (1).

19. L'article absorbant (1) selon la revendication 18, dans lequel les manchettes (6) de droite et de gauche ont des portions de base et dans lequel les poches de droite et de gauche sont disposées à l'intérieur des portions de base des manchettes (6) de droite et de gauche, respectivement.

20. L'article absorbant (1) selon l'une quelconque des revendications précédentes, dans lequel le noyau absorbant a la forme d'un sablier avec des portions d'extrémités élargies séparées par une portion centrale plus étroite, et dans lequel la portion centrale est plus épaisse que la portion d'extrémité.

21. L'article absorbant (1) selon la revendication 20, dans lequel la portion centrale comporte en outre de la mousse de sphaigne.

22. Un article absorbant (1) selon la revendication 1 et adapté pour emploi en liaison avec un sous-vêtement dont la portion d'entrejambe présente des bords de droite et de gauche, l'article absorbant (1) comportant :
a) des moyens de jonction pour joindre la première et la seconde couches, formant ainsi des bordures de droite et de gauche adjacentes aux côtés de droite et de gauche du noyau absorbant (7), respectivement ; dans lequel chaque manchette (6) est attachée, le long de sa portion de base respective, à l'article absorbant près des bordures de droite et de gauche et dans lequel l'extrémité distale de chaque manchette (6) s'en étend à l'extérieur, ce par quoi les extrémités distales des manchettes (6) sont, en service, comprimées contre une portion du corps de l'utilisateur ;
b) des ailes de droite et de gauche (19), présentant chacune une portion de base et une portion d'extrémité distale, la portion de base étant attachée à la couche imperméable aux fluides à l'intérieur de bords latéraux de l'article absorbant, formant ainsi des poches de droite et de gauche, la portion d'extrémité distale étant adaptée pour se replier et retenir les bords de droite et de gauche de la portion d'entrejambe du sous-vêtement dans les poches de droite et de gauche, respectivement.

23. L'article absorbant (1) selon la revendication 22, dans lequel la distance entre les poches de droite et de gauche est inférieure à la largeur de l'entrejambe du sous-vêtement.

24. L'article absorbant (1) selon la revendication 22 ou 23, dans lequel :
a) la première et la seconde couche (8, 9) ont chacune un premier et un second bords s'étendant approximativement longitudinalement ;
b) les premiers moyens de jonction comportent un premier adhésif disposé (i) entre une portion de la première couche (8), près de son bord de droite, et une portion de la seconde couche (9), près de son bord de droite et (ii) entre une portion de la première couche (8) près de son bord de gauche et une portion de la seconde couche (9) près de son bord de gauche ; et
c) les seconds moyens de jonction comportent un second adhésif disposé (i) entre la portion de la seconde couche (9), près de son bord de droite, et la portion de base de l'aile de droite (9) et (ii) entre la portion de la seconde couche (9), près de son bord de gauche, et la portion de base de l'aile de gauche (19), le premier et le second adhésifs étant disposés sur des surfaces opposées, respectivement, des portions de la seconde couche (9), près de ses bords de droite et de gauche.

25. L'article absorbant (1) selon l'une quelconque des revendications 22 à 24, dans lequel les portions de base des manchettes (6) de droite et de gauche sont disposées à l'extérieur des poches de droite et de gauche, respectivement.

26. L'article absorbant (1) selon l'une quelconque des revendications 22 à 25, dans lequel les côtés de droite et de gauche du noyau absorbant (7) sont disposés à l'extérieur des poches de droite et de gauche, respectivement, et dans lequel les bords de droite et de gauche de la portion d'entrejambe du sous-vêtement créent des forces s'exerçant vers le haut, directement sur la portion centrale, pour maintenir l'article absorbant (1) en position de contact avec le corps au cours de son emploi.

27. L'article absorbant (1) selon l'une quelconque des revendications 22 à 26, dans lequel les manchettes (6) de droite et de gauche comportent en outre un premier et un second ruban de matériau enclosant au moins une portion des bordures de droite et de gauche, respectivement.

28. L'article absorbant selon la revendication 27, dans lequel les portions de base des ailes (19) de droite et de gauche sont attachées à la seconde couche (9), par les bordures de droite et de gauche, les bases formant une portion des manchettes (6), et dans lequel le premier et le second rubans enclosent au moins une portion des bases des ailes (19) de droite et de gauche, respectivement.

29. Un article absorbant (1) selon la revendication 1 dans lequel :
a) la première couche (8) présente des bords s'étendant approximativement longitudinalement à droite et à gauche et la seconde couche (9) présente des bords s'étendant approximativement longitudinalement à droite et à gauche et dans lequel la première couche (8) est jointe à la seconde couche (9) près de leurs bords de droite et de gauche respectifs de façon à former des bordures de droite et de gauche, dans lequel les bordures de droite et de gauche sont adjacentes aux côtés latéraux de droite et de gauche de l'article absorbant (1);
b) chacune des manchettes (6) comporte une bande longitudinale du matériau élastique (47), perméable aux fluides, fortement élastique et poreux, et une bande longitudinale d'un film polymère perforé (48) qui enclôt le matériau densité basse, et épais le long d'au moins une portion substantielle de sa longueur et dans lequel les manchettes (6) s'étendant longitudinalement à droite et à gauche (30) sont fixées, le long de leurs portions de base respectives, aux côtés latéraux de droite et de gauche (30) de l'article absorbant (1) ;
c) des ailes de droite et de gauche, présentant chacune une portion de base et une portion d'extrémité distale, sont attachées, à leurs portions de base respectives, aux bordures de droite et de gauche, respectivement, et en formant une partie, les extrémités distales pouvant se replier par-dessus une portion de l'entrejambe du sous-vêtement d'un utilisateur.

30. L'article absorbant (1) selon la revendication 29, dans lequel le noyau absorbant (7) a la forme d'un sablier avec des portions d'extrémité élargies séparées par une portion centrale plus étroite et dans lequel la portion centrale est plus épaisse que la portion d'extrémité.

31. L'article absorbant selon la revendication 30 dans lequel la portion centrale comporte en outre de la mousse de sphaigne.
